# EUROPEAN PATENT SPECIFICATION

(11) **EP 4 480 377 B1**
(45) Date of publication and mention of the grant of the patent: **21.10.2026**
(21) Application number: 22950384.2
(22) Date of filing: 16.12.2022
(51) Int. Cl.: A47L 23/20, F26B 21/00, F26B 3/353, H01R 13/04, H01R 13/10

(54) **SHOE CARE DEVICE AND SHOE CARE SYSTEM**
SCHUHPFLEGEVORRICHTUNG UND SCHUHPFLEGESYSTEM
DISPOSITIF DE SOINS DE CHAUSSURE ET SYSTÈME DE SOINS DE CHAUSSURE

(30) Priority: 04.07.2022 KR 20220082009
(43) Date of publication of application: 25.12.2024
(73) Proprietor: LG Electronics Inc., Yeongdeungpo-gu Seoul 07336 (KR)
(72) Inventor: CHOI, Kyoung Min, Seoul 08592 (KR); LEE, Young Jae, Seoul 08592 (KR)
(74) Representative: Schornack, Oliver
(86) International application number: PCT/KR2022/020524
(87) International publication number: WO 2024/010159

(56) References cited:
- CN-A- 102 114 251
- KR-A- 20060 004 121
- KR-A- 20060 004 121
- KR-A- 20060 124 832
- KR-A- 20080 109 511
- KR-A- 20170 002 888
- KR-A- 20190 057 737
- KR-A- 20210 030 856
- US-A1- 2018 127 150

## Description

### [Technical Field]

The present invention relates to a shoe care device and more particularly, to a shoe care device that manages and displays shoes stored therein.

### [Background Art]

Shoes may get wet by a wearer's sweat, external contaminants, or rain or snow. Wearing such shoes may make the wearer uncomfortable, and in the condition, germs may also breed or stink in the shoes.

Accordingly, there is an increasing interest in a shoes care device, which removes germs and odors by performing a predetermined treatment on the shoes so that a user can comfortably wear the shoes all the times.

For the shoes care device, Korean Patent No. 1037245 (hereinafter, referred to as "Prior Document 1") discloses "Apparatus for sterilization disposal of shoes", which includes a main body, an ultraviolet emission module, and a deodorization module.

In addition, Korean Patent Unexamined Publication No. 10-2000-0009653 (hereinafter referred to as "Prior Document 2") discloses "The shoes cabinet for sanitation", which includes a main body, a far infrared radiation part, a circulation fan, an air circulation passage, and a sanitary filter portion.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device according to the Prior Documents 1 and 2 cannot properly solve such a task.

In recent years, people who collect shoes of popular brands as part of hobby or financial tech have appeared, and a show case has been disclosed which is configured to keep and display the shoes.

US Patent Unexamined Publication No. 2018-0127150 (hereinafter, referred to as 'Prior Document 3') discloses "modular storage container", and the modular storage container includes a housing, a door panel, a fan, and a light source.

In that Prior Document 3 above does not consider a technology of maintaining the internal temperature and humidity of the container in an optimal state, there is a risk of deformation or contamination of the shoe in the process of using the container by a user.

Typical users use or own multiple pairs of shoes, and for proper care of multiple shoes and step-by-step care of each pair of shoes, two or more devices for caring for shoes (shoe care devices) may be used together.

For example, one shoe care device may be used to remove odors or contaminants from shoes, and another shoe care device may be used to maintain the conditions of clean shoes (e.g., new shoes or shoes from which contaminants have been removed) for a long time.

As another example, different shoe care devices may be used together to care for different shoes.

In order to use two or more shoe care devices, power should be supplied to each shoe care device, and an effective and stable power connection is required.

US Patent Application Publication No. US 2018/0127150 A1 discloses a modular and stackable storage container. The modular storage container includes a housing, a contained volume defined within the housing, and a door panel. The modular storage container includes one or more accessories for providing additional functionality to the container including at least one fan, a light, and a heating element. The accessories are electrically powered by a base assembly. Further, the modular storage container includes a female connector positioned on a top end of the container being configured to engage a male connector of a container when another container is stacked above it.

### [Technical Problem]

In view of foregoing, an objective of the present invention is to provide a shoe care device that has a structure configured to receive external power as well as to supply power to another shoe care device, and the electrical connection of the two or more shoe care devices can be made easily and neatly.

In addition, an objective of the present invention is to provide a shoe care device that is configured to prevent the risk of electric shock.

Furthermore, an objective of the present invention is to provide a shoe care device , wherein all of the electrical connection portions of the shoe care device are configured to ensure effective waterproof, and the electrical connection of the two or more shoe care devices can be made easily.

### [Technical Problem]

A solution for aforementioned technical objectives is provided by a shoe care device according to claim 1. A shoe care device described herein may include an accommodation space for storing shoes, and may include an outer cabinet, a blower unit, a power supply unit, an inlet unit, and an outlet unit.

The outer cabinet defines the external appearance of the shoe care device.

The blower unit may be configured to circulate air in the accommodation space.

The power supply unit may be configured to supply power to the blower unit.

The inlet unit is electrically connected to the power supply unit and configured such that external power enters therethrough.

The outlet unit is electrically connected to the power supply unit and configured such that power goes out therethrough.

The inlet unit and the outlet unit may be provided on the rear surface of the outer cabinet.

The inlet unit is exposed on one side of the outer cabinet.

The shoe care device includes a flip (flap).

The flap is coupled to the outer cabinet and configured to shield or expose the outlet unit.

The shoe care device may include an outlet hole.

The outlet hole is a hole that penetrates the outer cabinet. The outlet unit may be exposed to the outside through the outlet hole.

The flap may be hinge-coupled to the outer cabinet at the edge of the outlet hole.

The shoe care device may include a flip spring (flap spring).

The flap spring may be configured to elastically support the flap in a direction in which the flap shields the outlet hole.

The flap and the flap spring may be located inside the outer cabinet.

When the outer surface of the flap is pressed inward, the outlet hole may be opened.

The shoe care device may include a first flip(first flap), a second flip(second flap), a first flip spring(first flap spring), and a second flip spring(second flap spring).

The first flap may be hinge-coupled to the outer cabinet at a first point on the edge of the outlet hole.

The second flap may be hinge-coupled to the outer cabinet at a second point located opposite to the first point on the edge of the outlet hole.

The first flap spring may be configured to elastically support the first flap in a direction in which the first flap shields the outlet hole.

The second flap spring may be configured to elastically support the second flap in a direction in which the second flap shields the outlet hole.

The width of the outlet unit in a first width direction may be longer than a width of the outlet unit in a second width direction perpendicular to the first width direction.

The first point and the second point may be spaced apart from each other along the second width direction.

The first flap, the second flap, the first flap spring, and the second flap spring may be located inside the outer cabinet.

The outlet hole may be opened when each of the outer surfaces of the first and second flaps is pressed inward.

The outer cabinet may include a depression.

The depression forms a portion recessed inward from the outer surface of the outer cabinet.

The outlet unit may be provided inside the outer cabinet at the point where the depression is formed.

The shoe care device may include a power cord device.

The power cord device may include an inlet connector that is detachably coupled to the inlet unit.

The shoe care device may include a connection cord device.

The connection cord device may include a first connector, a second connector, and a cable.

The first connector is detachably coupled to the outlet unit.

The second connector is detachably coupled to the inlet unit.

The cable is configured to electrically interconnect the first connector and the second connector.

The first connector may be configured to be coupled to the outlet unit, but not coupled to the inlet unit.

When the first connector is coupled to the outlet unit, a pin of the outlet unit moves outward to be electrically connected to a terminal of the first connector.

The first connector may include a push rib.

The push rib extends outward from the first connector.

The outlet unit may include a fixed bracket, a movable bracket, a slider, and a lever.

The fixed bracket is located inside the outer cabinet.

The movable bracket may be configured to be movable relative to the fixed bracket while the pin of the outlet unit is fixed.

The slider is configured to be moved by being pressed by the push rib.

The lever is coupled to the fixed bracket to be rotatable in opposite directions, and opposite ends of the lever are coupled to the movable bracket and the slider, respectively.

A pair of push ribs may be provided and arranged symmetrically with respect to the center of the first connector.

A pair of sliders and a pair of lever may be provided and arranged symmetrically with respect to the center of the outlet unit.

The outlet unit may include an outlet housing.

The outlet housing is configured to accommodate the fixed bracket, the movable bracket, the slider, and the lever, and is provided with a housing slit into which the push rib is inserted.

When the first connector is not coupled to the outlet unit, the pin of the outlet unit may be located inside the outlet housing.

When the first connector is coupled to the outlet unit, the pin of the outlet unit may penetrate the outlet housing and protrude outside the outlet housing.

The outer cabinet may include a connector slot.

The connector slot may be a hole formed through the outer cabinet such that the first connector is inserted thereinto. The connector slot may be provided in the form of a gap of which the width is narrow compared to its length.

The shoe care system described herein may include two or more shoe care devices and a connection cord device.

The shoe care devices each include an accommodation space configured to accommodate shoes, an operation unit configured to adjust at least one of the temperature, humidity, and air flow in the accommodation space, and a power supply unit configured to supply power to the operation unit.

The connection cord device may be configured to electrically interconnect a first shoe care device and a second shoe care device such that power from the power supply unit of the first shoe care device, which is one of the shoe care devices, is supplied to the power supply unit of the second shoe care device, which is another shoe care device.

Each of the first shoe care device and the second shoe care device include the inlet unit and the outlet unit.

The first connector may be detachably coupled to the outlet unit of the first shoe care device.

The second connector may be detachably coupled to the inlet unit of the second shoe care device.

Each of the first shoe care device and the second shoe care device may include an outer cabinet, a flap, and a flap spring. The outer cabinet, the flap, and the flap spring may each be formed as described above.

The outlet hole may be opened when the outer surface of the flap is pressed inward by the first connector.

### [Advantageous Effect]

In the shoe care device according to an embodiment of the present invention, the shoe care device may include an outer cabinet, a blower unit, a power supply unit, an inlet unit, and an outlet unit, the power cord device is electrically connected to one shoe care device, and two or more shoe care devices are electrically connected to each other by the connection cord device. According to the embodiment of the present invention, power can be supplied to two or more shoe care devices via one power cord device, and electrical connection of two or more shoe care devices can be made easily and neatly.

In the shoe care device according to an embodiment of the present invention, the shoe care device may include a flap and a flap spring. The flap may be hinge-coupled to the outer cabinet at the edge of the outlet hole, and the flap spring may be configured to elastically support the flap in the direction in which the flap shields the outlet hole. According to the embodiment of the present invention, since the outlet unit is shielded by the flap when the outlet unit is not coupled to the first connector, it is possible to prevent the risk of electric shock in all shoe care devices when a single shoe care device is used and when two or more shoe care devices are used together.

In the shoe care device according to an embodiment of the present invention, the shoe care device may include a first flap, a second flap, a first flap spring, and a second flap spring. The first flap is hinge-coupled to the outer cabinet at a first point on the edge of the outlet hole, and the second flap is hinge-coupled to the outer cabinet at a second point on the edge of the outlet hole. The width of the outlet unit in the first width direction is longer than the width of the second width direction perpendicular to the first width direction, and the first point and the second point may be spaced apart from each other along the second width direction. According to the embodiment of the present invention, the length from the outlet hole to the outlet unit can be made relatively shorter, and the first connector and the outlet unit are easily coupled. In addition, when two or more shoe care devices are used together, all of the shoe care devices are effectively waterproofed.

Additional effects and detailed effects of the shoe care device according to the embodiments of the present invention will be described below with reference to the attached drawings.

### [Brief Description of Drawings]

FIG. 1 is a perspective view illustrating a shoe care system according to an embodiment of the present disclosure.
FIG. 2 is a view illustrating a state in which a door is opened in a first shoe care device according to an embodiment of the present invention.
FIG. 3 is a perspective view illustrating the first shoe care device 1 of FIG. 2 viewed in another direction in the state in which the door and a first outer cabinet are partially removed.
FIG. 4 is a perspective view illustrating the first shoe care device of FIG. 3 viewed in another direction, in which a portion of a machine room is illustrated.
FIG. 5 is a view illustrating a connection relationship between respective components and the flow of fluid in the first shoe care device according to an embodiment of the present invention.
FIG. 6A is a perspective view illustrating the state in which shoes are accommodated in a second accommodation space of a second shoe care device according to an embodiment of the present invention.
FIG. 6B is a perspective view illustrating the second accommodation space of the second shoe care device of FIG. 6a in an open state.
FIG. 7 is a cross-sectional view illustrating a main body of the second shoe care device according to an embodiment of the present invention when an upper body is viewed from above.
FIG. 8 is a perspective view illustrating a shoe care system according to an embodiment of the present disclosure.
FIG. 9 is a view illustrating a power cord device according to an embodiment of the present invention.
FIGS. 10A and 10B are views each illustrating a connection cord device according to an embodiment of the present invention.
FIGS. 11A, 11B, and 11C are views each illustrating a shoe care system according to an embodiment of the present disclosure.
FIG. 12 is a view illustrating the internal appearance of an outer cabinet in a shoe care device according to an embodiment of the present invention in the state in which an outlet unit is installed.
FIG. 13 is a view illustrating a cross-sectional shape of a portion of a shoe care device according to an embodiment of the present invention, in which an outer cabinet, an outlet unit, and a flap are illustrated.
FIG. 14 is a view illustrating the flap in FIG. 13 in an open state.
FIG. 15A is a cross-sectional view of the shoe care device in the state in which the flap is closed.
FIG. 15B is a cross-sectional view of the shoe care device in the state in which the flap is opened.
FIG. 16 is a view illustrating the internal appearance of an outer cabinet in a shoe care device according to an embodiment of the present invention in the state in which a first connector and an outlet unit are coupled.
FIG. 17 is a view illustrating a connection cord device according to an embodiment of the present invention.
FIG. 18 is a view illustrating a first connector of the connection cord device according to an embodiment of the present invention.
FIG. 19 is a view illustrating the first connector and the outlet unit of FIG. 16 in a separated state, in which each component of the outlet unit is illustrated in a disassembled state.
FIG. 20A is a cross-sectional view illustrating the state in which the first connector and the outlet unit are separated from the shoe care device according to an embodiment of the present invention.
FIG. 20B is a cross-sectional view illustrating the state in which the first connector and the outlet unit in FIG. 20A are coupled.

### [Modes for the Invention]

Hereinafter, embodiments disclosed in this specification will be described in detail with reference to the accompanying drawings and the same or similar components are denoted by the same or similar reference numerals, and duplicated description thereof will be omitted. Suffixes "module" and "unit" for components used in the following description are given or mixed in consideration of easy preparation of the present invention only and do not have their own distinguished meanings or roles. Further, in describing the embodiment disclosed in this specification, a detailed description of related known technologies will be omitted if it is determined that the detailed description makes the embodiment disclosed in this specification unclear. Further, it is to be understood that the accompanying figures are just used for easily understanding the embodiments disclosed in this specification and a scope disclosed in this specification is not limited by the accompanying figures and all changes, equivalents, or substitutes included in the technical scope of the present invention are included.

Terms including an ordinary number, such as first and second, are used for describing various elements, but the elements are not limited by the terms. The terms are used only to discriminate one element from another element.

It should be understood that, when it is described that a component is "connected to" or "accesses" another component, the component may be directly connected to or access the other component or a third component may be present therebetween. In contrast, when it is described that a component is "directly connected to" or "directly accesses" another component, it is understood that no element is present between the element and another element.

A singular form includes a plural form if there is no clearly opposite meaning in the context.

In the present application, it should be understood that term "include" or "have"indicates that a feature, a number, a step, an operation, a component, a part or the combination thereof described in the specification is present, but does not exclude a possibility of presence or addition of one or more other features, numbers, steps, operations, components, parts or combinations thereof, in advance.

The present invention discloses a management device. Here, the management device may be divided into a first care device and a second care device.

The first device 1 may be configured to manage an article such that the article is kept clean or the physical/chemical conditions of the article are improved while the article is accommodated therein.

The second management device 1000 may be configured to manage an article such that the conditions of the article are maintained while the article is accommodated therein and may also be configured to manage the article such that the article is visible from the outside.

The present disclosure discloses a management system 2000. The management system 2000 may refer to a system that includes a plurality of first management devices 1, a plurality of second management devices 1000, or a system that includes at least one first management device 1 and at least one second management device 1000.

The articles described herein may include one or more of shoes, wallets, bags, dolls, toys, plastic models, accessories, and souvenirs. That is, the first management device 1 may be configured to manage one or more of shoes, wallets, bags, dolls, toys, plastic models, accessories, and souvenirs, and the second management device 1000 may be configured to manage one or more of shoes, wallets, bags, dolls, toys, plastic models, accessories, and souvenirs.

The shoes described in the present invention can be replaced with any one or more of wallets, bags, dolls, toys, plastic models, accessories, and souvenirs.

The first shoe care device described herein may be the first management device configured to care for shoes among the above-mentioned articles, the second shoe care device may be the second manager configured to manage shoes among the above-mentioned articles, and the shoe care system may be the management system configured to care for shoes among the above-mentioned articles.

The shoe care device described herein may be replaced by the first management device and/or the second management device.

The first shoe care device described herein may be replaced with the first management device, the second shoe care device may be replaced with the second management device, and the shoe care system may be replaced with the management system.

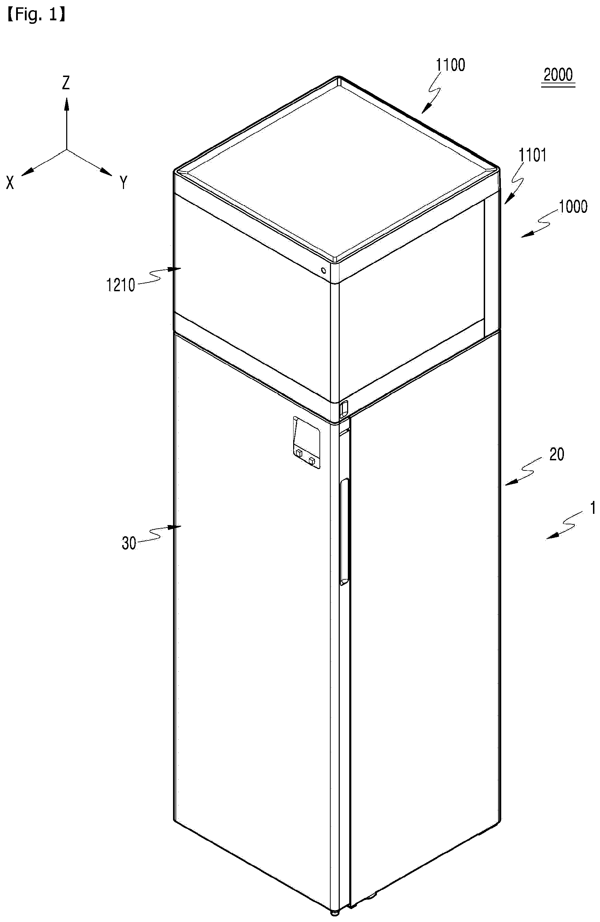

FIG. 1 is a perspective view illustrating a shoe care system 2000 according to an embodiment of the present disclosure.

A first direction X, a second direction Y, and a third direction Z described in the embodiment of the present invention may be directions orthogonal to each other.

The first direction X and the second direction Y may be directions parallel to a horizontal direction, and the third direction Z may be a direction parallel to a vertical direction. When the first direction X is a direction parallel to a left and right direction, the second direction Y may be a direction parallel to a front and rear direction. When the first direction X is a direction parallel to a front and rear direction, the second direction Y may be a direction parallel to a left and right direction.

The shoe care system 2000 according to an embodiment of the present disclosure may include two or more shoe care devices 1 and 1000.

The shoe care devices 1 and 1000 according to an embodiment of the present invention may be divided into a first shoe care device 1 and a second shoe care device 1000.

The first shoe care device 1 and the second shoe care device 1000 include a part that is in common with each other. Unless otherwise specifically limited, a "shoe care device" described in an embodiment of the present invention may be a "first shoe care device" or a "second shoe care device".

A plurality of shoe care devices 1 and 1000 may be arranged adjacent to each other. The first shoe care device 1 and the second shoe care device 1000 may be stacked in a vertical direction. A plurality of second shoe care devices 1000 may be stacked vertically.

In an embodiment of the present invention, the first shoe device 1 may be used to remove odors or contaminants from shoes, and the second shoe care device 1000 may be used to maintain the conditions of currently clean shoes (e.g., new shoes or shoes from which contaminants have been removed) for a long time.

The shoe care device 1 or 1000 according to an embodiment of the present invention includes an accommodation space 101 or 1010, an outer cabinet 20 or 1101, and an operation unit 2700. The shoe care device 1 or 1000 may include a blowing part 310 or 1330.

The outer cabinet 20 or 1101 defines the external appearance of the shoe care device 1 or 1000.

The accommodation space 101 or 1010 is the internal space of the shoe care device 1 or 1000 configured to accommodate shoes therein.

The operation unit 2700 is configured to adjust one or more of the temperature, humidity, and air flow in the accommodation spaces 101 and 1010.

The blowing parts 310 and 1330 may be configured to circulate the air in the accommodation spaces 101 and 1010.

Each shoe care device 1 or 1000 according to an embodiment of the present invention may include a controller 10 or 1600. The controller 10 or 1600 is configured to control the operation of each shoe care device 1 or 1000. The controller 10 or1600 may be configured to process a signal, data, information, or the like input to or output from the shoe care device, and may include memory configured to store application programs for driving each component of the shoe care device.

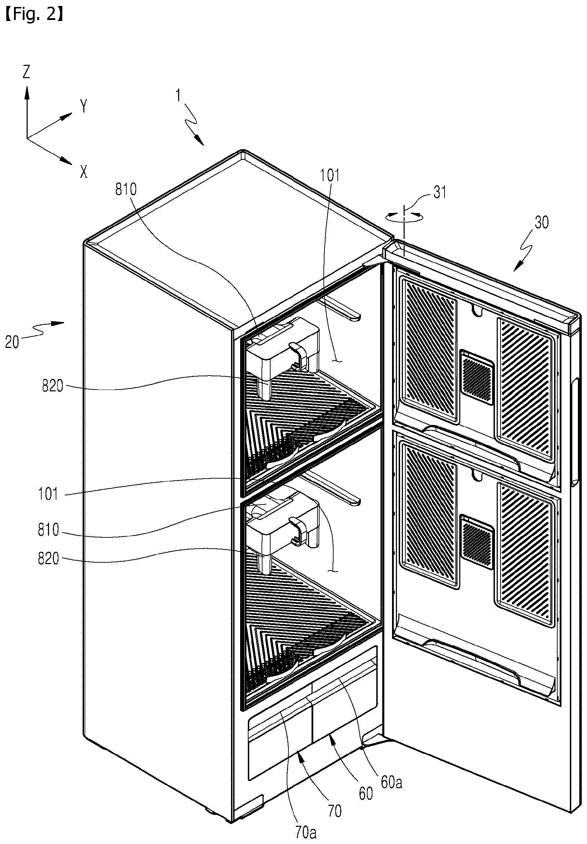

FIG. 2 is a view illustrating the state in which the door 30 is opened in the first shoe care device 1 according to an embodiment of the present invention.

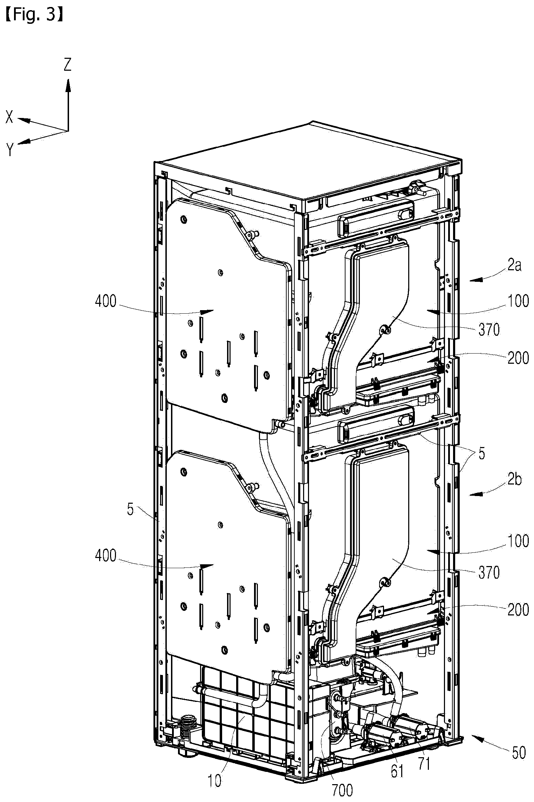

FIG. 3 is a perspective view illustrating the first shoe care device 1 of FIG. 2 viewed in another direction in the state in which the door 30 and the first outer cabinet 20 are partially removed.

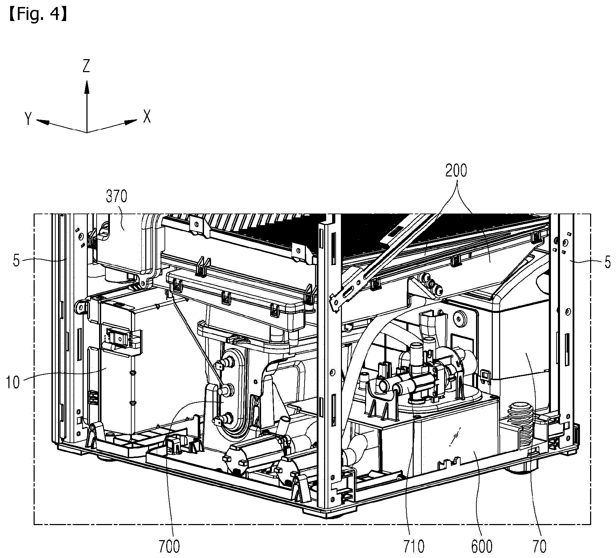

FIG. 4 is a perspective view illustrating the first shoe care device 1 of FIG. 3 viewed in another direction, in which a portion of a machine room 50 is illustrated.

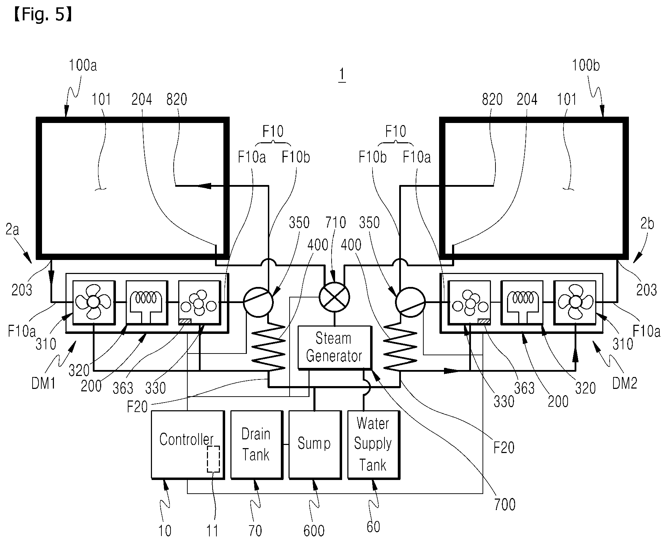

FIG. 5 is a view illustrating the connection relationship between respective components and the flow of fluid in the first shoe care device 1 according to an embodiment of the present invention.

A first shoes care device 1 according to one embodiment of the present invention may include an outer cabinet 20, a door 30, an inner cabinet 100, and a machine room 50. The first shoes care device 1 may include a frame 5.

In one embodiment of the present invention, the outer cabinet 20 of the first shoes care device 1 may refer to the first outer cabinet 20.

The first outer cabinet 20 and the door 30 may form an overall appearance of the first shoes care device 1. The outside of the first shoes care device 1 may be formed in a hexahedral shape. That is, while the first outer cabinet 20 and the door 30 are coupled to each other and the door 30 is closed, the appearance of the first shoes care device 1 may be formed in a hexahedral shape. However, the first shoes care device 1 according to one embodiment of the present invention is not limited to such a shape and may have various three-dimensional shapes.

The main frame 5 may form an overall framework of the first shoes care device 1. The main frame 5 may have a hexahedral structure.

The first outer cabinet 20 may be detachably fixed to the frame 5.

The door 30 is configured to open and close the inside of the first shoes care device 1. The door 30 may form any one surface of the first shoes care device 1. The door 30 may form a left side or a right side of the shoes care device 1, or may form a front side of the first shoes care device 1.

In one embodiment, the door 30 may be hinge-coupled to the main frame 5. In another embodiment, the door 30 may be hinge-coupled to the outer cabinet 20, and in another embodiment, the door 30 may be hinge-coupled to the inner cabinet 100 and/or the machine room 50.

A hinge rotation axis 31 of the door 30 may be formed in the vertical direction. That is, in the shoes care device 1, the door 30 may be configured to be rotatable bidirectionally around a rotation axis 31 in the vertical direction.

Hereinafter, except for a particularly limited case, it will be described that the door 30 is formed in a front side of the first shoes care device 1. That is, a surface on which the door 30 is formed in the first shoes care device 1 is described as a front surface of the first shoes care device 1.

Furthermore, hereinafter, except for a particularly limited case, it will be described that the first direction (X direction) is parallel to the front-rear direction, the second direction (Y direction) is parallel to the left-right direction, and the third direction (Z direction) is parallel to the vertical direction.

The inner cabinet 100 and the machine room 50 may be provided inside the outer cabinet 20.

The inner cabinet 100 may be formed in a box shape, and a predetermined space may be formed therein. The space inside the inner cabinet 100 forms an accommodation space 101, and shoes S may be accommodated in the accommodation space 101.

In an embodiment of the present invention, an accommodation space of the first shoe care device 1 is referred to as the first accommodation space 101.

A plurality of shoes S may be disposed together in the first accommodation space 101 of one inner cabinet 100.

The inner cabinet 100 may be fixed to the main frame 5.

The inner cabinet 100 is formed in the shape of a box opened to any one side. The inner cabinet 100 may be formed in a form opened to the front side of the first shoes care device 1. The opening of the inner cabinet 100 may be closed or opened by the door 30.

In one embodiment, the machine room 50 may be provided in a lower side of the inner cabinet 100. In the machine room 50, some of the components that constitutes the first shoes care device 1 may be accommodated, and, in this case, the components that are accommodated in the machine room 50 may be fixed to a frame 5 or to the inner cabinet 100 or the outer cabinet 20.

The first shoes care device 1 according to one embodiment of the present invention includes management devices 2a and 2b. In the shoes care device 1, a plurality of management devices 2a and 2b may be provided. In one embodiment, the first shoes care device 1 may include a first management device 2a and a second management device 2b. That is, the first shoes care device 1 may include two separate management devices 2a and 2b.

The 'management device' described in the present invention may refer to a 'first management device 2a' and a 'second management device 2b', respectively, except for a particularly limited case.

The management devices 2a and 2b include the above-described inner cabinet 100.

In one embodiment of the present invention, since the inner cabinet 100 of the first management device 2a and the inner cabinet 100 of the second management device 2b may be distinguished from each other, the inner cabinet 100a of the first management device 2a may refer to a first inner cabinet 100a, and inner cabinet 100a of the second management device 100b may refer to a second inner cabinet 100b.

It may be understood that the 'inner cabinet 100' described in one embodiment of the present invention refers to each of the 'first inner cabinet 100a' and the 'second inner cabinet 100b', except for a particularly limited case.

When the door 30 is closed in the shoes care device 1, the door 30 closes the main opening of the first management device 2a and also closes the main opening of the second management device 2b. That is, the door 30 may simultaneously seal the first accommodation space 101 of the inner cabinet 100 of the first management device 2a and the first accommodation space 101 of the inner cabinet 100 of the second management device 2b. In this case, the first accommodation space 101 of the inner cabinet 100 of the first management device 2a and the first accommodation space 101 of the inner cabinet 100 of the second management device 2b may be configured such that they do not communicate with each other.

As described above, in one embodiment of the present invention, the first accommodation space 101 of the inner cabinet 100 of the first management device 2a and the first accommodation space 101 of the inner cabinet 100 of the second management device 2b may form independent spaces, and may form blocked spaces (not communicating with each other). Accordingly, the temperature and humidity of the first accommodation space 101 of the first management device 2a and the temperature and humidity of the first accommodation space 101 of the second management device 2b may be controlled differently from each other.

The management devices 2a and 2b may be configured to include a connection path F10. The management devices 2a and 2b may be configured to include a blowing part 310 and a dehumidifying part 330. The management devices 2a and 2b may include an outlet 203 and a nozzle 820.

In one embodiment of the present invention, the blowing part 310 of the first shoe care device 1 may refer to the first blowing part 310.

The management devices 2a and 2b may include the module housing 200 forming a connection path F10. The module housing 200 forms all or part of the connection path F10

The management devices 2a and 2b may include a regeneration path F20. The management devices 2a and 2b may include a heating part 320.

In one embodiment of the present invention, the heating part 320 of the first shoe care device 1 may refer to the first heating part 320.

The management devices 2a and 2b may include a conversion flow path F10a.

The management devices 2a and 2b may include a damper 350.

The first shoes care device 1 may include a steam generator 700 and a steam valve 710.

The first shoes care device 1 may include a sump 600, a water supply tank 60, and a drain tank 70.

The connection path F10 forms a flow path of a fluid.

The connection path F10 forms a passage through which air and/or condensed water inside the first shoes care device 1 moves.

The dehumidifying part 330 is disposed inside the connection path F10 and includes a dehumidifying material 331. The dehumidifying part 330 may be entirely formed of the dehumidifying material, or a part thereof may be formed of the dehumidifying material. A further explanation of the dehumidifying part 330 will be described below.

According to one embodiment of the present invention, the first shoes care device 1 has an air circulation structure in which the air inside the inner cabinet 100 in which the shoes are disposed is sucked into the connection path F10 to dehumidify the air using a dehumidifying material 331 and the dehumidified air may be supplied back into the inner cabinet 100.

The connection path F10 may be used as a means to achieve such an air circulation structure in the first shoes care device 1. All or part of the connection path F10 may be formed of a pipe, a hose, a tube, a duct, a housing, or a combination thereof.

The module housing 200 according to one embodiment of the present invention forms part of the connection path F10.

The outlet 203 is formed in the module housing 200. The outlet 203 communicates with the first accommodation space 101 of the inner cabinet 100, and forms an inlet of the module housing 200 through which the air of the first accommodation space 101 of the inner cabinet 100 is suctioned into the module housing 200.

In the shoes care device 1, the inner cabinet 100 and the machine room 50 may form a space separated from each other. Furthermore, the module housing 200 that is part of the management devices 2a and 2b may be provided between the inner cabinet 100 and the machine room 50.

In the first shoes care device 1 according to one embodiment of the present invention, the first blowing part 310, the dehumidifying part 330 (and the dehumidifying material 331), and the first heating part 320 may be accommodated inside the module housing 200.

In addition, the machine room 50 may be configured to accommodate a controller 10, the sump 600, the steam generator 700, and the steam valve 710 therein. Furthermore, the machine room 50 may be configured to accommodate the water supply tank 60 and the drain tank 70.

In one embodiment of the present invention, the controller 10 of the first shoe care device 1 may refer to a first controller 10.

Among the components constituting the management devices 2a and 2b, components not included in the module housing 200 may be fixedly coupled to the inner cabinet 100 and the outside of the module housing 200 or may be fixedly coupled to the frame 5.

Components coupled to or accommodated in the machine room 50 may be fixedly coupled to the machine room 50.

Each of the water supply tank 60 and the drain tank 70 may be formed in the form of a container for accommodating water.

The water supply tank 60 may be configured to store water supplied into the first shoes care device 1 inside. The water supply tank 60 may be configured to store water supplied into the steam generator 700 inside. In order to supply water from the water supply tank 60 into the shoes care device 1, a water pump 61 may be connected to the water supply tank 60.

The drain tank 70 may be configured to store water discharged from the first shoes care device 1 inside. The drain tank 70 may store water condensed inside the first shoes care device 1. The drain tank 70 may be configured to store water drained from the sump 600. In order to discharge water to the drain tank 70, a water pump 71 (a second water pump) may be connected to the drain tank 70.

The water supply tank 60 and the drain tank 70 may be coupled to the machine room 50 to get exposed from the outside of one wall surface of the machine room 50.

The water supply tank 60 and the drain tank 70 may be configured to be detachable from the machine room 50. In order to facilitate the attachment and detachment of the water supply tank 60 and the drain tank 70, a handle 60a of the water supply tank 60 may be formed on an outer surface of the water supply tank 60, and a handle 70a of the drain tank 70 may be formed on an outer surface of the drain tank 70.

The first controller 10 may be configured to control operations of each component in connection with each component constituting the first shoes care device 1.

In order to control the controller 10, the first shoes care device 1 may be provided with a storage medium in which an application program is stored, and the first controller 10 may be configured to control the first shoes care device 1 by driving an application program according to information input to the first shoes care device 1 and information output from the first shoes care device 1.

The first controller 10 may control the first management device 2a and the second management device 2b constituting the first shoes care device 1 to operate individually. The first controller 10 may control the first management device 2a and the second management device 2b to operate in different states, and may control shoes (e.g., sneakers) in the first management device 2a and shoes (e.g., heels) in the second management device 2b to be managed under different conditions. Furthermore, the first controller 10 may control the first management device 2a and the second management device 2b to interwork with each other.

The first shoes care device 1 is provided with the steam generator 700 as a device configured so as to generate moisture inside the inner cabinet 100. The steam generator 700 is configured to generate steam and selectively supply moisture and steam to the inside of the inner cabinet 100.

Moist air formed by the steam generator 700 is supplied toward the first accommodation space 101 of the inner cabinet 100, and moisture may be circulated in the first accommodation space 101 of the inner cabinet 100, and accordingly, the moisture may be supplied to the shoes S.

The first shoes care device 1 according to one embodiment of the present invention may be a refresher device that refreshes the shoes.

Here, refreshing may refer to a process of removing contaminants, deodorizing, sanitizing, preventing static electricity, or warming by supplying air, heated air, water, mist, steam, etc. to the shoes.

The steam generator 700 may supply steam to the first accommodation space 101 of the inner cabinet 100 in which the shoes S are accommodated, and may perform steam treatment on the shoes, which is further meant to exert a refreshing effect due to swelling of shoes materials as well as sterilization by high-temperature steam.

The steam generator 700 may generate steam by receiving water from the water supply tank 60.

The steam valve 710 is configured to be connected to the steam generator 700. The steam valve 710 is configured to selectively communicate with each of the first accommodation space 101 of the first management device 2a and the first accommodation space 101 of the second management device 2b, respectively.

When the steam of the steam generator 700 is supplied to the first accommodation space 101 of the first management device 2a and/or the first accommodation space 101 of the second management device 2b, the steam valve 710 operates to control whether the stream is supplied or not, and an operation of the stream 710 is driven by the first controller 10.

In the first shoes care device 1 according to one embodiment of the present invention, the dehumidifying part 330 may be used as a means that dehumidifies air.

The dehumidifying part 330 may be configured to be porous by itself. A plurality of pores may be formed over an entire volume of the dehumidifying part 330, and air may move by penetrating the dehumidifying part 330 through such pores.

The dehumidifying part 330 may be formed of dehumidifying materials, and may be configured to include the dehumidifying materials.

The dehumidifying material 331 according to one embodiment of the present invention is configured to include a material capable of reducing humidity by absorbing moisture in the air. The dehumidifying material 331 may be formed of various materials or a combination of the materials within the range of absorbing or adhering the moisture in the air, and may be formed in various shapes and structures.

The dehumidifying material according to one embodiment of the present invention may be referred to as a desiccant or an adsorbent.

The dehumidifying material according to one embodiment of the present invention may be formed of a microporous material. The dehumidifying material according to one embodiment of the present invention may include silica gel, activated carbon, activated alumina (AL2O3), diatomaceous earth, etc.

Specifically, the dehumidifying material according to one embodiment of the present invention may be formed of zeolite or may be configured to include zeolite.

The first blowing part 310 is provided inside the connection path F10. A blowing fan may be provided inside the first blowing part 310. Since the first blowing part 310 is provided in the connection path F10, air flows in the connection path F10 when the first blowing part 310 is driven, and since the connection path F10 also communicates with the first accommodation space 101 of the inner cabinet 100, the air flows and moves in the first accommodation space 101 by driving the first blowing part 310.

In this way, the air may be sucked from the inner cabinet 100 into the connection path F10 by the driving of p the first blowing part 310 (a rotation of the blowing fan 313), and the air inside the connection path F10 may be ventilated.

In one embodiment, the first blowing part 310 is provided inside the module housing 200 forming the connection path F10, and the first blowing part 310 is driven to suction the air inside the inner cabinet 100 into an inlet 203. The air inside the connection path F10 passes through the module housing 200 constituting the connection path F10, the dry air duct 370, and a nozzle duct 810 and is then discharged back into the inner cabinet 820.

As such, the flow of air may be generated in the first shoes care device 1 by the driving of the first blowing part 310.

The first heating part 320 is provided at one side of the dehumidifying part 330 and the first blowing part 310 in the connection path F10. The first heating part 320 may be provided inside the module housing 200. Based on a movement direction of the air inside the module housing 200, the module housing 200 may be arranged in an order of the first blowing part 310, the first heating part 320, and the dehumidifying part 330. That is, the air introduced into the outlet 203 of the module housing 200 moves along the connection path F10 by passing sequentially through the first blowing part 310, the first heating part 320, and the dehumidifying part 330.

The first heating part 320 is configured to heat the air of the module chamber 210 inside the module housing 200.

The first heating part 320 may be configured to heat the dehumidifying part 330.

The air heated by the first heating part 320 by the driving of the first blowing part 310 moves directly to the dehumidifying part 330, thus heating the dehumidifying part 330. To this end, the first heating part 320 is disposed inside the module housing 200 adjacent to the dehumidifying part 330. Specifically, the first heating part 320 is disposed inside the module housing 200 adjacent to the dehumidifying part 330 based on a movement path of the air inside the module housing 200.

In the module housing 200, the dehumidification by the dehumidifying material or the regeneration of the dehumidifying material may be achieved by selectively heating the first heating part 320.

The first heating part 320 may be made up of various devices and structures within a range capable of heating the air inside the module housing 200 or supplying heat to the dehumidifying part 330. The first heating part 320 may be formed of an electric heater 321.

In one embodiment of the present invention, the first blowing part 310, the first heating part 320, the dehumidifying part 330, and the module housing 200 may form one set. The set may be provided in a plural form. The first shoes care device 1 according to one embodiment may be provided with two sets. Such a set may be provided in each of the first management device 2a and the second management device 2b.

Such a set may form a drying module DM in the first shoes care device 1 according to one embodiment of the present invention.

That is, in one embodiment of the present invention, the drying module DM may include the module housing 200, the first blowing part 310, the first heating part 320, and the dehumidifying part 330. Furthermore, the drying module DM is provided in each of the first management device 2a and the second management device 2b.

In the first shoes care device 1 according to one embodiment of the present invention, a plurality of drying modules DM may be provided.

In the first shoes care device 1 according to one embodiment of the present invention, a pair of drying modules DM may be provided. When the first shoes care device 1 is provided with the pair of drying modules DM, one of the drying modules DM may form a 'drying module A (DM1) ' as a drying module of the first management device 2a, and the other may form a 'drying module B (DM2) ' as a drying module of the second management device 2b.

The 'drying module' described in one embodiment of the present invention may be understood to refer to each of the 'drying module A' and the 'drying module B' except as otherwise particularly limited.

In the first shoes care device 1 according to one embodiment of the present invention, the drying module A (DM1) and the drying module B (DM2) may operate in different modes. When the drying module A DM1 operates in a moisture absorption mode, the drying module B DM2 may operate in a regeneration mode. Conversely, when the drying module A DM1 operates in the regeneration mode, the drying module B DM2 may operates in the moisture absorption mode.

The 'moisture absorption mode' described in the present invention means a case in which the dehumidifying part 330 adsorbs moisture in the air, and the 'regeneration mode' means a case in which the moisture adsorbed to the dehumidifying part 330 is separated by heating the dehumidifying part 330.

Naturally, both the drying module A DM1 and the drying module B DM2 may operate in the moisture absorption mode or may operate in the regeneration mode.

The first shoes care device 1 according to one embodiment of the present invention includes the condenser 400 coupled to an outer surface of the inner cabinet 100 and forming a regeneration path F20.

In the first shoes care device 1 according to one embodiment of the present invention, steam generated by the steam generator 700 is supplied to the first accommodation space 101 of the inner cabinet 100, and to this end, the first shoes care device 1 includes a steam inlet 204.

The steam inlet 204 forms an inlet through which steam is supplied to the first accommodation space 101 of the inner cabinet 100.

The connection path F10 forms a movement path of air connected from the outlet 203 to the nozzle 820. That is, the outlet 203 may form an inlet of the connection path F10, and the nozzle 820 may form an outlet of the connection path F10.

The connection path F10 may be divided into a conversion flow path F10a and a drying flow path F10b. The conversion flow path F10a and the drying flow path F10b form a movement path of air sequentially connected to each other. The air in the connection path F10 may sequentially move through the conversion flow path F10a and the drying flow path F10b.

The conversion flow path F10a forms an upstream section of the connection path F10, which is connected to the outlet 203. The conversion flow path F10a may be a section in which the first blowing part 310, the first heating part 320, and the dehumidifying part 330 are disposed. The conversion flow path F10a may be formed by the module housing 200, and the module chamber 210 inside the module housing 200 may form the conversion flow path F10a.

The conversion flow path F10a may be a section in which humid air moves and dries. The conversion flow path F10a may be a section in which air is dehumidified by the dehumidifying part 330.

Meanwhile, the conversion flow path F10a may be a section in which the dehumidifying part 330 are regenerated.

The drying flow path F10b forms a downstream section of the connection path F10, which connects the conversion flow path F10a to the nozzle 820. A flow path formed by the drying air duct 370, the nozzle duct 810, and the nozzle 820 may form the drying flow path F10b.

The drying passage F10b may be a section in which dry air with moisture removed therefrom moves.

When the drying module DM operates in the moisture absorption mode, the drying flow path F10b communicates with the conversion flow path F10a, and when the drying module DM operates in the regeneration mode, the drying flow path F10b and the conversion flow path F10a may not communicate with each other such that the drying flow path F10b and the conversion flow path F10a block each other.

Accordingly, when air is dehumidified by the dehumidifying part 330 in the conversion flow path F10a, the dried air moves through the drying flow path F10b.

As described above, after humid air in the first accommodation space 101 of the inner cabinet 100 flows into the conversion flow path F10a, the air is dehumidified by the dehumidifying part 330 and converted into dry air, and the dry air can be resupplied to the first accommodation space 101 of the inner cabinet 100 through the drying flow path F10b.

The regeneration path F20 forms a movement path of a fluid.

The regeneration path F20 forms a passage through which air and/or condensed water inside the shoes care device moves.

The regeneration path F20 forms a path through which air and/or condensed water passing through the dehumidifying part 330 moves when the dehumidifying part 330 is regenerated. The regeneration path F20 may be entirely or partially formed of a pipe, a hose, a tube, a duct, a housing, or a combination thereof.

Moisture generated during the regeneration process of the dehumidifying part 330 needs to be discharged through a separate flow path separated from the drying flow path F10b, which is a flow path through which dry air moves. Accordingly, the first shoes care device 1 according to one embodiment of the present invention includes the regeneration path F20, and when the dehumidifying material is regenerated, air passing through the dehumidifying part 330 is not ventilated to the nozzle 820 but moves through the regeneration path F20.

The regeneration path F20 is a flow path branched from the connection path F10. The regeneration path F20 may be branched from the connection path F10 to form a path different from that of the drying flow path F10b of the connection path F10. The regeneration path F20 is connected to the sump 600.

The regeneration path F20 may be a section that connects the conversion flow path F10a and the sump 600.

The regeneration path F20 may be a section in which humid air separated from the dehumidifying part 330 moves.

The condenser 400 according to one embodiment of the present invention forms a regeneration path F20. Moisture separated from the dehumidifying material 331 may be condensed after moving to the condenser 400 along with air moving along the regeneration path F20. In addition, condensed water condensed in the condenser 400 may be moved to the sump 600 through the regeneration path F20, collected from a lower part of the sump 600, and then discharged to the drain tank 70, discharged to the outside, or pressed to the steam generator 700.

In the first shoes care device 1 according to one embodiment of the present invention, when the drying module DM operates in the regeneration mode, the regeneration path F20 communicates with the conversion flow path F10a, and when the drying module DM operates in the moisture absorption mode, the regeneration path F20 and the conversion flow path F10 may not communicate with each other such that the regeneration path F20 and the conversion flow path F10 block each other.

Accordingly, when the dehumidifying part 330 is regenerated in the conversion flow path F10a, humid air containing moisture separated from the dehumidifying part 330 moves through the regeneration path F20.

In one embodiment of the present invention, the damper 350 may be formed in the form of a damper valve.

The damper 350 controls a movement path of air passing through the dehumidifying material in the module housing 200. Depending on the operation of the damper 350, the air passing through the dehumidifying material may move into the inner cabinet 100 through the nozzle 820 or may move into the regeneration path F20.

The damper 350 may be configured to open the regeneration path F20 while blocking the drying flow path F10b, or to open the drying flow path F10b while blocking the regeneration path F20.

Referring to FIG. 5, in the drying module A (DM1) of the first management device 2a, the damper 350 seals the wet air outlet 232 and opens the dry air outlet 231 of the second management device 2b, and in the drying module B (DM2) of the second management device 2b, when the damper 350 opens the wet air outlet 232 and seals the dry air outlet 231, the air in the conversion flow path F10a of the first management device 2a may flow through the drying flow path F10b, and the air in the conversion flow path F10a of the second management device 2b may flow through the regeneration path F20. Furthermore, in this case, the drying module A (DM1) of the first management device 2a may operate in the moisture absorption mode, and the drying module B (DM2) of the second management device 2b may operate in the regeneration mode.

In contrast, when in the drying module A (DM1) of the first management device 2a, the damper 350 opens the wet air outlet 232 and seals the dry air outlet 231, and in the drying module B (DM2) of the second management device 2b, the damper 350 seals the wet air outlet 232 and opens the dry air outlet 231, the air in the conversion flow path F10a of the first management device 2a may flow along the regeneration path F20, and the air in the conversion flow path F10a of the second management device 2b may flow along the drying flow path F10b. Furthermore, in this case, the drying module A (DM1) of the first management device 2a may operate in the regeneration mode, and the drying module B (DM2) of the second management device 2b may operate in the moisture absorption mode.

In both the drying module A (DM1) of the first management device 2a and the drying module B (DM2) of the second management device 2b, when the damper 350 seals the wet air outlet 232 and opens the dry air outlet 231, both the drying module A DM1 and the drying module B (DM2) may operate in the moisture absorption mode.

In both the drying module A (DM1) of the first management device 2a and the drying module B (DM2) of the second management device 2b, when the damper 350 seals the dry air outlet 231 and opens the wet air outlet 232, both the drying module A (DM1) and the drying module B (DM2) may operate in the regeneration mode.

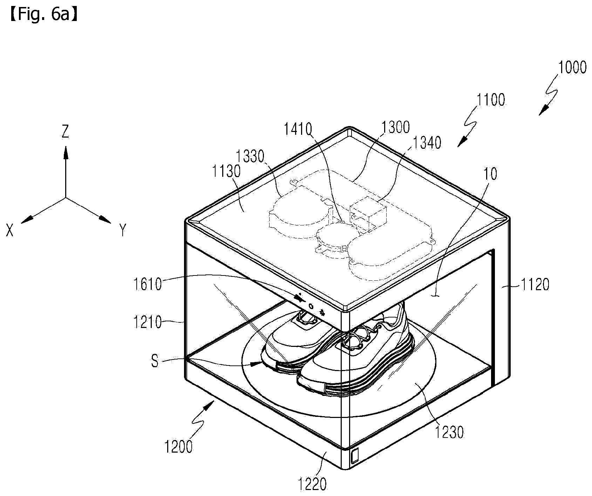

FIG. 6A is a perspective view illustrating the second shoe care device 1000 according to an embodiment of the present invention in the state in which shoes S are accommodated in the second accommodation space 1010.

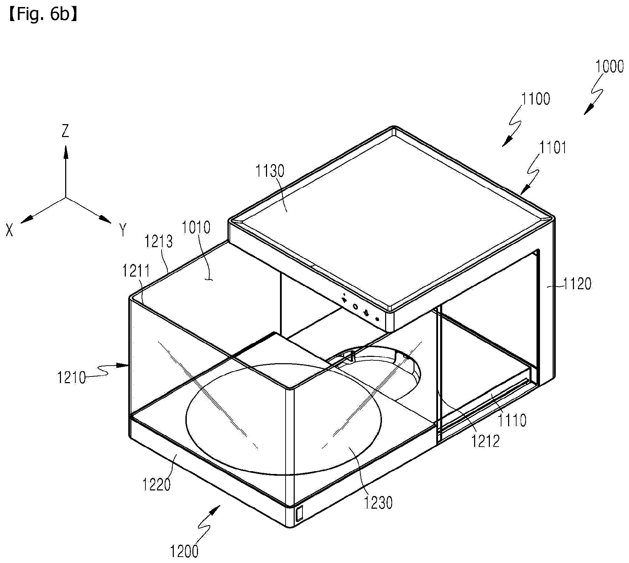

FIG. 6B is a perspective view illustrating the second accommodation space 1010 of the second shoe care device 1000 of FIG. 6A in an open state.

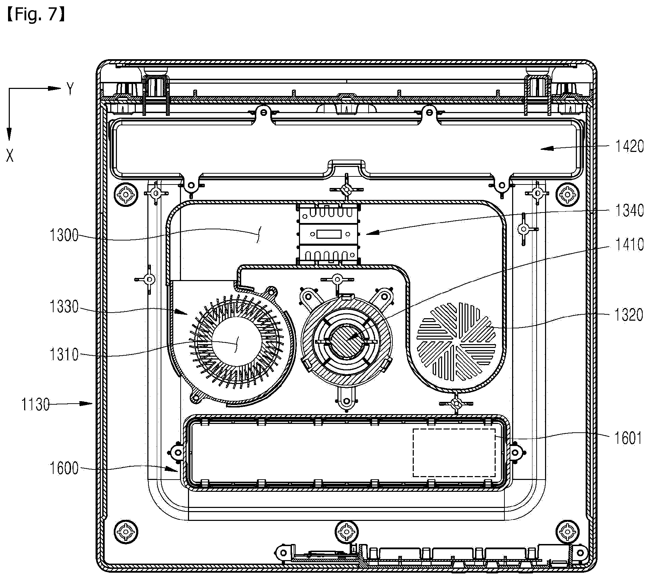

FIG. 7 is a cross-sectional view illustrating a main body 1100 of the second shoe care device 1000 according to an embodiment of the present invention, with an upper body 1130 viewed from above.

The second shoe care device 1000 according to an embodiment of the present invention is configured to safely store shoes, to form and adjust the individual environments required for shoes (e.g., temperature, humidity, and the like in predetermined ranges), to effectively display the shoes, and to increase user convenience.

As illustrated in FIGS. 6A and 6B, the second shoe care device 1000 according to an embodiment of the present invention includes a main body 1100 and a movable body 1200.

The main body 1100 and the movable body 1200 together form an accommodation space 1010 configured to accommodate shoes S.

In an embodiment of the present invention, the accommodation space of the second shoe care device 1000 will be referred to as a second accommodation space 1010.

The main body 1100 and the movable body 1200 are coupled to each other to enable relative movement. The movable body 1200 may be coupled to the main body 1100 to be reciprocally movable in the horizontal direction.

The main body 1100 defines the outer cabinet 1101 of the second shoe care device 1000. The outer surface portion of the main body 1100 may define the outer cabinet 1101 of the second shoe care device 1000.

In an embodiment of the present invention, the outer cabinet of the second shoe care device 1000 may be referred to as a second outer cabinet 1101.

The movable body 1200 may slide in the first direction X relative to the main body 1100, and the second accommodation space 1010 may be opened while the second shoe care device 1000 is transformed from the closed state to the open state.

The second shoe care device 1000 illustrated in FIG. 6B may be changed as illustrated in FIG. 6A. That is, the movable body 1200 may slide in the first direction X relative to the main body 1100, and the second accommodation space 1010 may be closed while the second shoe care device 1000 is transformed from the closed state to the open state.

As such, in the second shoe care device 1000 according to an embodiment of the present invention, the movable body 1200 is movable in a first direction X or in a direction opposite to the first direction X relative to the main body 1100, and is reciprocally movable forward and rearward.

When the second accommodating space 1010 is closed, the second accommodating space 1010 may be sealed from external air. Therefore, when shoes S are accommodated in the second accommodation space 1010 and the second accommodation space 1010 is closed, the shoes S can be blocked from contact with dust and moisture in the external air.

The main body 1100 may define the upper and rear surfaces of the second accommodation space 1010.

The movable body 1200 may define the front surface, bottom surface, and opposite side surfaces of the second accommodation space 1010.

The second accommodation space 1010 may have a hexahedral shape. However, the second accommodation space 1010 of the second shoe care device 1000 according to an embodiment of the present invention is not limited to this shape and may have various three-dimensional shapes.

The main body 1100 and the movable body 1200 may define the overall external appearance of the second shoe care device 1000. The exterior of the second shoe care device 1000 may have a hexahedral shape. That is, in the state in which the main body 1100 and the movable body 1200 are coupled to each other and the second accommodation space 1010 is closed, the external appearance of the second shoe care device 1000 may have a hexahedral shape. However, the second shoe care device 1000 according to an embodiment of the present invention is not limited to this shape and may have various three-dimensional shapes.

The body 1100 may include an upper body 1130, a middle body 1120, and a lower body 1110.

The upper body 1130 is positioned at an upper side of the second accommodation space 1010. The upper body 1130 forms the upper surface of the second accommodation space 1010. The upper body 1130 may form an uppermost portion of the second shoe care device 1000. The upper surface of the upper body 1130 may form a flat surface along a substantially horizontal surface. When a plurality of shoe care devices 1 is provided, any one second shoe care device 1000 may be placed on the upper surface of the upper body 1130 of the other one second shoe care device 1000, and the shoe care devices 1 may be stacked on each other.

The lower body 1110 is positioned below the second accommodation space 1010. The lower body 1110 may form a lowermost portion of the second shoe care device 1000. The lower body 1110 may form a bottom portion of the second shoe care device 1000.

The middle body 1120 is positioned behind the second accommodation space 1010. The middle body 1120 forms the rear surface of the second accommodation space 1010. The middle body 1120 may connect the upper body 1130 and the lower body 1110 behind the second accommodation space 1010. The middle body 1120 may form a rear wall surface of the second shoe care device 1000.

The body 1100 is configured to include the upper body 1130, the middle body 1120, and the lower body 1110, and as a result, the body 1100 may form a substantially 'C' form on the side view.

As described above, the moving body 1200 is configured to move forward and backward with respect to the body 1100. The moving body 1200 may include a base 1220, a transparent window 1210, and a turntable 1230.

The base 1220 may be coupled to the lower body 1110 to be slidably movable in the first direction X. The base 1220 may form the bottom portion of the moving body 1200. The base 1220 may be positioned at the upper side of the lower body 1110. A bottom surface of the base 1220 may be positioned in close contact with or in proximity to the upper surface of the lower body 1110.

The transparent window 1210 may form of extending upward from the base 1220. The transparent window 1210 may form the front surface, and both side surfaces (a left surface and a right surface) of the second accommodation space 1010. The transparent window 1210 may be made of a transparent or translucent material.

Light inside and outside the second accommodation space 1010 may pass through the transparent window 1210. The transparent window 1210 may be made of a material which is weather-resistant to prevent discoloration. The transparent window 1210 may be made of an acrylic (PMMA) material which is weather-resistant and scratch-resistant.

The transparent window 1210 may prevent a beam having a predetermined wavelength from being introduced into the second accommodation space 1010. As an example, the transparent window 1210 may be configured to block ultraviolet rays. The ultraviolet rays as an electromagnetic wave in which a wavelength corresponds to 10 to 397 nm shorter than visible rays are light which has a strong chemical action and causes getting sunburn or discoloration.

As an example, an ultraviolet-proof film may be attached to an inner surface or an outer surface of the transparent window 1210. Alternatively, the inner surface or the outer surface of the transparent window 1210 may be UV-coated with an ultraviolet-proof agent.

The transparent window 1210 includes a first window 1211, a second window 1212, and a third window 1213. The first window 1211 may form the front surface of the second accommodation space 1010. The second window 1212 may form the left surface of the second accommodation space 1010. The third window 1213 may form the right surface of the second accommodation space 1010.

In the state in which the shoe S is accommodated in the second accommodation space 1010, a user may view the shoe S through the transparent window 1210. As a result, the second shoe care device 1000 may be used as a device which may display the shoe S while keeping and caring the shoe S.

As illustrated in FIG. 6a, in the state in which the moving body 1200 is positioned relatively at a rearmost side, the second accommodation space 1010 may be sealed from the outside air. In this case, the second accommodation space 1010 may be formed in the hexahedral form. In this case, the moving body 1200 may be present at a first location and the second shoe care device 1000 is in the closed state.

As illustrated in FIG. 6b, in the state in which the moving body 1200 moves in the first direction X, the second accommodation space 1010 may be opened. In this case, an upper portion of the first window 1211 is spaced to a front side in the first direction X from the front surface of the upper body 1130 to form a gap (hereinafter, referred to as 'first gap').

In this case, the second window 1212 is spaced to a front side in the first direction X from the left side surface of the middle body 1120 to form a gap (hereinafter, referred to as 'second gap'). The third window 1213 is spaced to the front side in the first direction X from a right side surface of the middle body 1120 to form a gap (hereinafter, referred to as 'third gap').

In the state in which the moving body 1200 moves in the first direction X as much as possible, i.e., in the state in which the moving body 1200 is positioned relatively at a frontmost side, the moving body 1200 may be present at a second location and the second shoe care device 1000 may be in the opened state.

The user may put the shoe S into the second accommodation space 1010 or withdraw the shoe S from the second accommodation space 1010 through the first gap.

The turntable 1230 may form the upper surface on which the shoe S is placed. The upper surface of the turntable 1230 may have a circular shape. The turntable 220 may form the lower surface of the second accommodation space 1010 jointly with the base 1220.

The turntable 1230 may be rotatably coupled to the base 1220 around a vertical axis, i.e., an axis parallel to a third direction Z.

For the rotation of the turntable 1230, a motor may be provided in the moving body 1200. The motor may be coupled to the base 1220. The turntable 1230 may rotate in conjunction with the rotation of the motor shaft. Rotational force of the motor may be delivered to the turntable 1230 through a reducer. The motor may rotate unidirectionally or reciprocally rotate bidirectionally.

The turntable 1230 is provided, and as a result, the shoe S may rotate in the second accommodation space 1010 or a display effect of the shoe S may be enhanced.

The second shoe care device 1000 may include an operating button 1610 and a controller 1600. The operating button 1610 may be formed in the body 1100. As an example, the operating button 1610 may be formed on the front surface of the upper body 1130. When the user manipulates the operating button 1610, the turntable 1230 may rotate or stop. The user manipulates the operating button 1610 to adjust a rotational speed of the turntable 1230. The user may input a rotation time of the turntable 1230 into the controller 1600 through the operating button 1610.

In an embodiment of the present invention, the controller of the second shoe care device 1000 is referred to as the second controller 1600.

The operating button 1610 may be formed in the body 1100. As an example, the operating button 1610 may be formed on the front surface of the upper body 1130. When the user manipulates the operating button 1610, the turntable 1230 may rotate or stop. The user manipulates the operating button 1610 to adjust a rotational speed of the turntable 1230. The user may input a rotation time of the turntable 1230 into the second controller 1600 through the operating button 1610.

The user manipulates the operating button 1610 to rotate the turntable 1230 at a predetermined angle. In the state in which the second accommodation space 1010 is opened, the user may place the shoe S on the upper surface of the turntable 1230 by holding any one part (heel top, lining, tong, etc.) of the shoe S. Thereafter, the user manipulates the operating button 1610 to rotate the turntable 1230 at a predetermined angle.

As an example, the user manipulates the operating button 1610 to rotate the turntable 1230 at a predetermined angle so that a front and rear direction of the shoe S coincides with the first direction X. Alternatively, the user manipulates the operating button 1610 to rotate the turntable 1230 at a predetermined angle so that the front and rear direction of the shoe S form a predetermined angle with the first direction X.

Therefore, even though the user places the shoe S on the upper surface of the turntable 1230 while gripping any one part of the shoe S, the shoe S may be placed (displaced) in a direction desired by the user.

The rotational speed of the turntable 1230 according to the shape, size, and/or type of the shoe S may be set in the second controller 1600. Alternatively, the user may input the rotational speed of the turntable 1230 according to the shape, size, and/or type of the shoe S into the second controller 1600 through the operating button 1610.

The second shoe care device 1000 according to the embodiment of the present invention may include a first light 1410. The first light 1410 may be provided in the upper body 1130.

The first light 1410 may illuminate the second accommodation space 1010. The first light 1410 may include a light source 1411 and a lens.

The light source of the first light 1410 may intensively irradiate light onto the upper surface of the turntable 1230 on which the shoe S is placed. The light of the light source may intensively illuminate the shoe S placed on the upper surface of the turntable 1230. When the first light 1410 is turned, an image of the shoe S stored in the second accommodation space 1010 may be changed by the light of the light source.

The light of the light source of the first light 1410 may illuminate the second accommodation space 1010 by passing through the lens. An ultraviolet-proof film may be attached to the lens or the ultraviolet-proof agent may be coated on the lens in order to block the ultraviolet rays.

When the user manipulates the operating button 1610, the light source may be turned on or off. The user may input an operating time of the light source into the second controller 1600 through the operating button 1610. The user manipulates the operating button 1610 to adjust the operating time of the light source.

The operating time and the operating pattern of the light source according to the shape, size, and/or type of the shoe S may be set in the second controller 1600. The user may input the operating time and the operating pattern of the light source according to the shape, size, and/or type of the shoe S into the second controller 1600 through the operating button 1610.

The body 1100 may be configured to include an air path 1300. The body 1100 may be configured to include a suction port 1310 and a discharge port 1320.

The air path 1300 may be provided inside the upper body 1130.

The air path 1300 connects the suction port 1310 and the discharge port 1320. The suction port 1310 may form an inlet of the air path 1300, and the discharge port 1320 may form an outlet of the air path 1300.

The suction port 1310 may be formed on a bottom surface of the upper body 1130. The discharge port 1320 may be formed on the bottom surface of the upper body 1130.

The air in the second accommodation space 1010 may be suctioned into the air path 1300 through the suction port 1310. The air in the air path 1300 may be discharged to the second accommodation space 1010 through the discharge port 1320. Therefore, air forcibly blown by a second blowing part 1330 to be described below may be circulated in the second accommodation space 1010 and the air path 1300.

The second shoe care device 1000 according to the embodiment of the present invention may include the blowing part 1330 and a heating part 1340.

In an embodiment of the present invention, the blowing part of the second shoe care device 1000 is referred to as the second blowing part 1330, and the heating part of the second shoe care device 1000 is referred to as the second heating part 1340.

The second blowing part 1330 and the second heating part 1340 may be provided in the upper body 1130.

The second blowing part 1330 is configured to circulate the air in the second accommodation space 1010.

The second blowing part 1330 may be placed in the air path 1300, and may generate a flow of the air in the air path 1300.

The second heating part 1340 may be configured to directly or indirectly heat the air in the second accommodation space 1010. The second heating part 1340 may be placed in the air path 1300.

The body 1100 may be configured to include a second light 1420. The second light 1420 may be configured to be formed on the bottom surface of the upper body 1130 and to illuminate the middle body 1120 behind the first light 410.

The second heating part 1340 may deliver thermal energy to the air which moves in the air path 1300. The heating part 340 may be configured to include a heat wire 341.

The second blowing part 1330 and the second heating part 1340 may control the temperature and/or humidity of the second accommodation space 1010.

When the user manipulates the operating button, the second blowing part 1330 may rotate or stop. The user manipulates the operating button to adjust the rotational speed of the second blowing part 1330. The user may input the rotation time of the second blowing part 1330 into the controller through the operating button. The air in the second accommodation space 1010 may be circulated by operating the second blowing part 1330, and the air in the second accommodation space 1010 may maintain a uniform state as a whole.

When the user manipulates the operating button, the second heating part 1340 may be turned on or off. The user manipulates the operating button to adjust the operating time of the second heating part 1340. The user may input an operating temperature of the second heating part 1340 into the second controller through the operating button. The temperature of the air heated by the second heating part 1340 may be selected or adjusted according to characteristics of a used shoe S.

The second blowing part 1330 and the second heating part 1340 may be operated for a predetermined time. Further, each of the operating and the stopping of the second blowing part 1330 and the second heating part 1340 may be repeatedly conducted for a predetermined time. The time may be decided by the user, or automatically decided by a program.

According to the embodiment of the present invention, the temperature and/or the humidity of the second accommodation space 1010 may be maintained at an optimal state which is suitable for the characteristics of each shoe S.

Therefore, the second shoe care device 1000 according to the embodiment of the present invention may achieve both a shoe display effect of displaying the shoe and a shoe care effect of interrupting deformation or contamination of the shoe.

Alternatively, the operating time and the operating pattern of the second blowing part 1330 and the second heating part 1340 according to the shape, size, and/.or type of the shoe may be set in the second controller 1600. The user may input the operating time and the operating pattern of the second blowing part 1330 and the second heating part 1340 according to the shape, size, and/.or type of the shoe into the second controller through the operating button.

Therefore, the second shoe care device 1000 according to the embodiment of the present invention controls the temperature and/or the humidity of the second accommodation space 1010 differently according to a material, a load, the shape, the size, and/or the type of the shoe to completely interrupt the deformation or the contamination of the shoe.

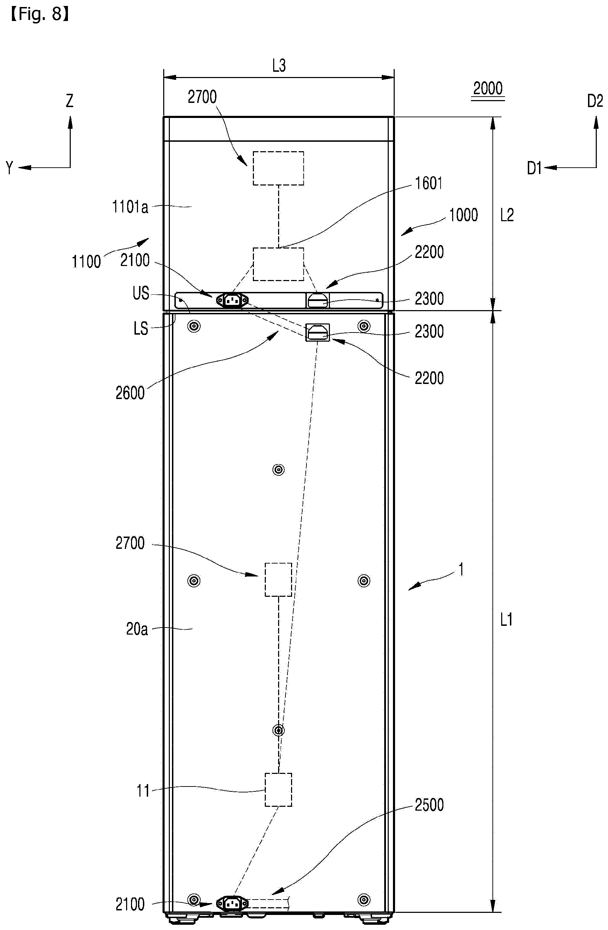

FIG. 8 is a view illustrating a shoe care system 2000 according to an embodiment of the present disclosure. In FIG. 8, a power cord device 2500 and a connection cord device 2600 are schematically shown with dotted lines, and the electrical connection relationship between respective components in the shoe care system 2000 is schematically shown with dotted lines.

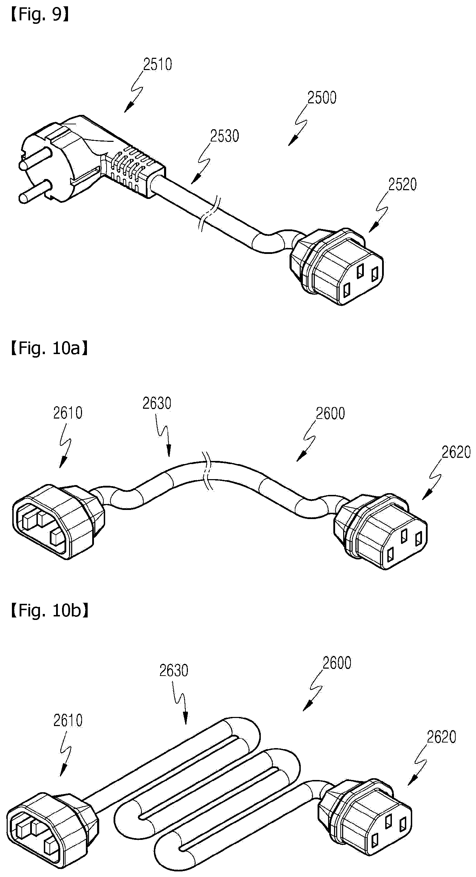

FIG. 9 is a view illustrating the power cord device 2500 according to an embodiment of the present invention.

FIGS. 10A and 10B are views each illustrating the connection cord device 2600 according to an embodiment of the present invention.

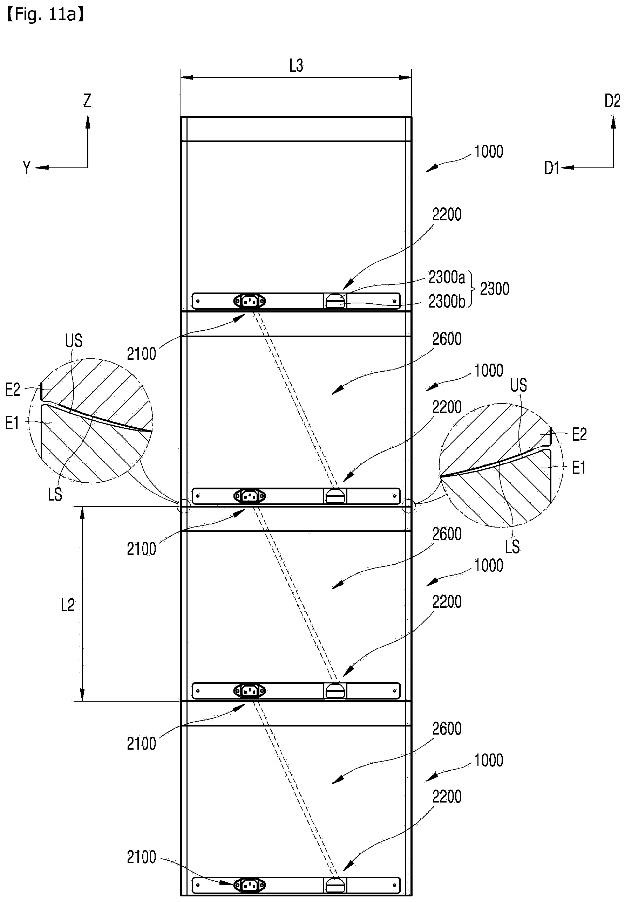

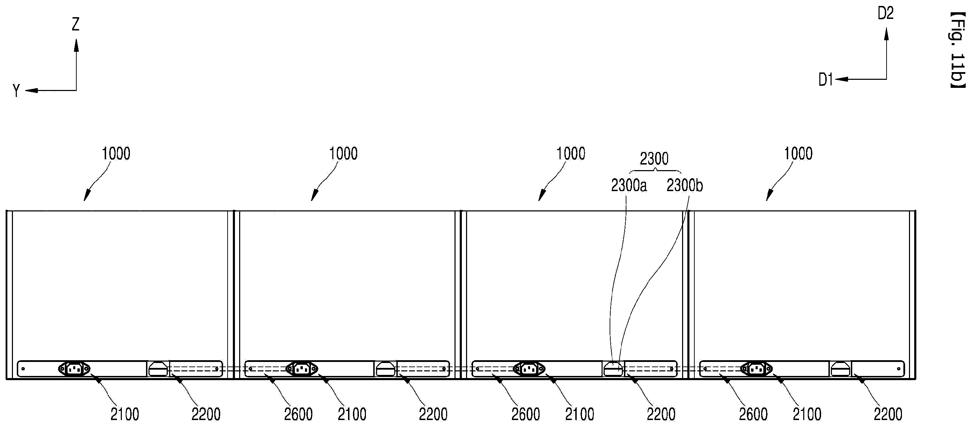

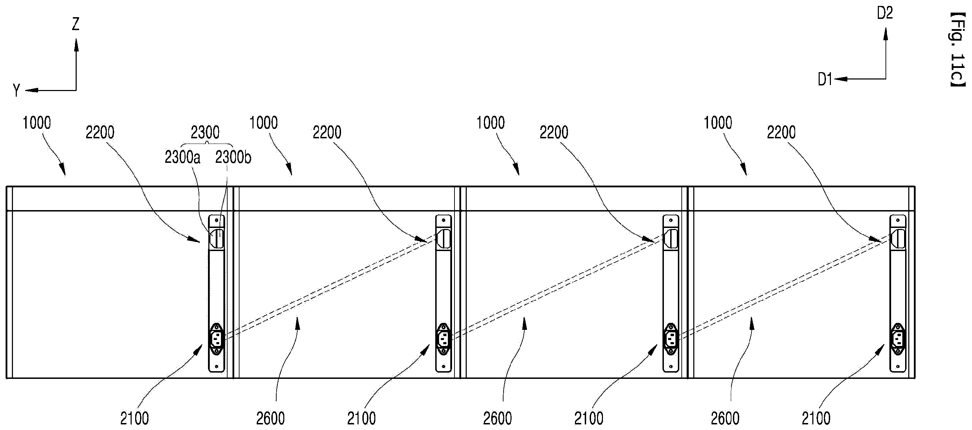

FIGS. 11A, 11B, and 11C are views each illustrating the shoe care system 2000 according to an embodiment of the present disclosure. In FIGS. 11A, 11B, and 11C, connection cord devices 2600 are schematically illustrated with dotted lines in the state in which each connection code device 2600 electrically interconnects two adjacent second shoe care devices 1000.

In each of FIGS. 11A, 11B, and 11C, outlet units 2200 are illustrated in the state of covered by flaps 2300. Of course, the flaps 2300 will be opened when the connection cord devices 2600 are coupled to the outlet units 2200.

The shoe care device 1 or 1000 according to an embodiment of the present invention includes a power supply unit 11 or 1601, an inlet unit 2100, and an outlet unit 2200.

Each of the inlet unit 2100 and the outlet unit 2200 according to an embodiment of the present invention is an electrical connection device. Each of the inlet unit 2100 and the outlet unit 2200 according to an embodiment of the present invention may be an electrical connector.

The first shoe care device 1 includes a power supply unit 11 (a first power supply unit), an inlet unit 2100, and an outlet unit 2200. The second shoe care device 1000 includes a power supply unit 1601 (a second power supply unit), an inlet unit 2100, and an outlet unit 2200.

The power supply unit 11 or 1601 is a power supply unit and may be configured as a device configured to supply electricity to each component of each shoe care device.

The power supply unit 11 or 1601 is configured to supply power to an operation unit 2700. The power supply unit 11 or 1601 may be configured to supply power to a blowing part 310 or 1330.

A first blowing part 310 and/or a first heating part 320 of the first shoe care device 1 may constitute the operating part 2700 of the first shoe care device 1.

A second blowing part 1330 and/or a second heating part 1340 of the second shoe care device 1000 may constitute the operating part 2700 of the second shoe care device 1000.

The power supply unit 11 or 1601 may be configured integrally with the controller 10 or 1600, or may be configured separately from the controller 10 or 1600. The power supply unit 11 or 1601 may form a part of the controller 10 or 1600.

The inlet unit 2100 is electrically connected to the power supply unit 11 or 1601. External power enters each shoe care device through the inlet unit 2100. By connecting the power supply unit 11 or 1601 to the inlet unit 2100, external power of the shoe care device can be supplied to each component of the shoe care device through the inlet unit 2100 and the power supply unit 11 or 1601.

The outlet unit 2200 is electrically connected to the power supply unit 11 or 1601. Power goes out from the shoe care device to the outside through the outlet unit 2200. By connecting the power supply unit 11 or 1601 to the outlet unit 2200, the power supplied to the inside of the shoe care device can be supplied to the outside of the shoe care device through the power supply unit 11 or 1601 and the outlet unit 2200. The power supplied to one shoe care device may be supplied to another shoe care device through the outlet unit 2200.

The inlet unit 2100 may be in the form of a socket or plug.

The outlet unit 2200 may be in the form of a socket or plug.

The inlet unit 2100 and the outlet unit 2200 may be made to meet each of the standards prescribed international organizations such as International Electrotechnical Commission (IEC), European Standard (IECEE (or CEE)), National Electrical Manufacturers Association (NEMA), and British Standards Institution (BSI), and may be made in various forms.

Each of the inlet unit 2100 and the outlet unit 2200 may be made in Type A (NEMA 1-15), Type B (NEMA 5-15), Type C (CEE 7/1), Type D, Type E (CEE 7/5), Type F (CEE 7/3, CEE 7/4), Type G (BS 1363), Type H, Type I, Type J, Type K, Type L, Type M, Type N, or Type O.

The inlet unit 2100 and the outlet unit 2200 may each be made as an AC terminal (IEC-320). The inlet unit 2100 and the outlet unit 2200 may each be made in the form of an IEC-320 (IEC_60320) terminal used to connect an AC power cable.

The inlet unit 2100 may be made to conform to the structure, shape, and specifications of a male terminal (IEC-320 C14).

The outlet unit 2200 may be made to conform to the structure, shape, and specifications of a female terminal (IEC-320 C13).

The inlet unit 2100 and the outlet unit 2200 may be provided on the outer surface 20a or 1101a of the outer cabinet 20 or 1101 or may be provided adjacent to the outer surface of the outer cabinet 20 or 1101.

The inlet unit 2100 and the outlet unit 2200 may be provided adjacent to each other.

The inlet unit 2100 and the outlet unit 2200 may be provided on the same surface of the outer cabinet 20 or 1101. The inlet unit 2100 and the outlet unit 2200 may be provided together on the rear surface 20a or 1101a of the outer cabinet 20 or 1101. Accordingly, the connection cord device 2600 to be used when two or more shoe care devices are disposed in succession (see FIGS. 8, 11A, 11B, and 11C) may have a relatively short length, the connection cord device 2600 and the power cord device 2500 may be arranged neatly, and each shoe care device may be electrically connected easily.

When the inlet unit 2100 and the outlet unit 2200 are provided on one surface (e.g., the rear surface 20a or 1101a) of the outer cabinet 20 or 1101, the inlet unit 2100 and the outlet unit 2200 may be disposed at various positions to be spaced apart from each other. In an embodiment, the inlet unit 2100 and the outlet unit 2200 may be arranged to be spaced apart from each other in the width direction (e.g., the horizontal direction), and in another embodiment, the inlet unit 2100 and the outlet unit 2200 may be arranged to be spaced apart from each other in the length direction (e.g., the vertical direction).

The inlet unit 2100 is coupled to one side of the outer cabinet 20 or 1101.

The shoe care device may include a power cord device 2500.

The power cord device 2500 is electrically coupled to the shoe care device to transmit external electrical energy into the shoe care device.

The power cord device 2500 may include an inlet connector 2520 that is detachably attached to the inlet unit 2100.

The power cord device 2500 may include a power connector 2510 and a cable 2530. The cable 2530 of the power cord device 2500 will be referred to as a "first cable 2530".

The power connector 2510 may be in the form of a male terminal (a plug). The power connector 2510 may be made to meet each of the standards prescribed international organizations such as International Electrotechnical Commission (IEC), European Standard (IECEE (or CEE)), National Electrical Manufacturers Association (NEMA), and British Standards Institution (BSI), and may be made in various forms.

The power connector 2510 may be made in Type A (NEMA 1-15), Type B (NEMA 5-15), Type C (CEE 7/1), Type D, Type E (CEE 7/5), Type F (CEE 7/3, CEE 7/4), Type G (BS 1363), Type H, Type I, Type J, Type K, Type L, Type M, Type N, or Type O.

The power connector 2510 may be coupled to another electrical connector or electrical socket to transmit electrical energy to the shoe care device.

The first cable 2530 is an electric cable that electrically interconnects the power connector 2510 and the inlet connector 2520.

A shoe care device may include a connection cord device 2600.

The connection cord device 2600 electrically interconnects one shoe care device to another shoe care device.

In an embodiment, the connection cord device 2600 transmits the electrical energy of the first shoe care device 1 to the second shoe care device 1000. The connection cord device 2600 may electrically interconnect the first shoe care device 1 and the second shoe care device 1000 such that power of the power supply unit 11 of the first shoe care device 1, which is one shoe care device, is supplied to the power supply unit 1601 of the second shoe care device 1000, which is another shoe care device.

In another embodiment, the connection cord device 2600 transmits the electrical energy of one second shoe care device 1000 to another second shoe care device 1000. The connection cord device 2600 may electrically interconnect second shoe care devices 1000 such that power of the power supply unit 1601 of one second shoe care device 1000 is supplied to the power supply unit 1601 of another second shoe care device 1000.

The connection cord device 2600 may include a first connector 2610, a second connector 2620, and a cable 2630. The cable 2630 of the connection cord device 2600 will be referred to as a "second cable 2630".

The second cable 2630 is an electric cable that electrically interconnects the first connector 2610 and the second connector 2620. The length of the second cable 2630 may vary depending on the size, shape, or the like of the shoe care devices to be used.

The first connector 2610 is detachably attached to the outlet unit 2200. The first connector 2610 may be detachably coupled to the outlet unit 2200 of the first shoe care device 1. The first connector 2610 may be detachably coupled to the outlet unit 2200 of the second shoe care device 1000.

The second connector 2620 is detachably attached to the outlet unit 2100. The first connector 2620 may be detachably coupled to the inlet unit 2100 of the second shoe care device 1000.

Each of the inlet connector 2520, the first connector 2610, and the second connector 2620 may be made to meet each of the standards prescribed international organizations such as International Electrotechnical Commission (IEC), European Standard (IECEE (or CEE)), National Electrical Manufacturers Association (NEMA), and British Standards Institution (BSI), and may be made in various forms.

Each of the inlet connector 2520, the first connector 2610, and the second connector 2620 may be made in Type A (NEMA 1-15), Type B (NEMA 5-15), Type C (CEE 7/1), Type D, Type E (CEE 7/5), Type F (CEE 7/3, CEE 7/4), Type G (BS 1363), Type H, Type I, Type J, Type K, Type L, Type M, Type N, or Type O.

The inlet connector 2520, the first connector 2610, and the second connector 2620 may each be made as an AC terminal (IEC-320). The inlet connector 2520, the first connector 2610, and the second connector 2620 may each be in the form of an IEC-320 (IEC_60320) terminal used when connecting an AC power cable.

Each of the inlet connector 2520 and the second connector 2620 has a corresponding shape, structure, and size to be capable of being fastened to the inlet unit 2100. When the inlet unit 2100 is made in the form of a female terminal, each of the inlet connector 2520 and the second connector 2620 is made in the form of a male terminal. When the inlet unit 2100 is made in the form of a male terminal, each of the inlet connector 2520 and the second connector 2620 is made in the form of a female terminal.

The inlet connector 2520 and the second connector 2620 may be made to conform to the structure, shape, and specifications of a female terminal (IEC-320 C13).

The first connector 2610 may have a corresponding shape, structure, and size to be capable of being fastened to the outlet unit 2200. When the outlet unit 2200 is made in the form of a female terminal, the first connector 2610 may be made in the form of a male terminal. When the outlet unit 2200 is made in the form of a male terminal, the first connector 2610 may be made in the form of a female terminal.

The first connector 2610 may be made to conform to the structure, shape, and specifications of a male terminal (IEC-320 C14).

The first connector 2610 may be configured to be coupled to the outlet unit 2200 but not coupled to the inlet unit 2100. The first connector 2610 and the outlet unit 2200 are made in forms of correspond to each other as a male and a female (e.g., in the form in which one is inserted into another one such that the two are tightly coupled to each other), and the first connector 2610 and the inlet unit 2100 may be made such that their sizes and shapes do not correspond to each other to ensure that the first connector and the inlet unit are not coupled to each other.

In a shoe care system 2000 according to an embodiment of the present disclosure, a first shoe care device 1 and a second shoe care device 1000 may be arranged in a vertically stacked form. For example, the first shoe care device 1 and the second shoe care device 1000 may be arranged in the form in which the second shoe care device 1000 is stacked on the first shoe care device 1. In this case, the power cord device 2500 may be connected and coupled to the inlet unit 2100 of the first shoe care device 1, and the connection cord device 2600 may be connected to the first shoe care device 1 and the second shoe care device 1000 (see FIG. 8).

In a shoe care system 2000 according to an embodiment of the present disclosure, a plurality of second shoe care devices 1000 may be arranged in a vertically stacked form. In this case, the power cord device 2500 may be connected and coupled to the inlet unit 2100 of the lowermost second shoe care device 1000, and the connection cord device 2600 may interconnect two second shoe care devices 1000 stacked vertically to be adjacent to each other (see FIG. 11A).

In a shoe care system 2000 according to an embodiment of the present disclosure, a plurality of second shoe care devices 1000 may be arranged to be in close contact with each other from side to side. In this case, the power cord device 2500 may be connected and coupled to the inlet unit 2100 of the leftmost or rightmost second shoe care device 1000, and the connection cord device 2600 may interconnect two second shoe care devices 1000 arranged to be adjacent to each other (see FIGS. 11B and 11C).

In an embodiment, the inlet units 2100 and the outlet units 2200 may be provided to be arranged along the horizontal direction on the rear surface of the second shoe care devices 1000 (see FIG. 11B). In addition, the inlet units 2100 and the outlet units 2200 may be provided on the lower sides of the second shoe care devices 1000. In this case, when two adjacent second shoe care devices 1000 are arranged in the horizontal direction, the outlet unit 2200 of one of the second shoe care devices 1000 and the inlet unit 2100 of the other second shoe care device 1000 are arranged relatively close together, and the length between the outlet unit 2200 of one second shoe care device 1000 and the inlet unit 2100 of the other second shoe care device 1000 is smaller than the horizontal width L3 of the second shoe care devices 1000. Therefore, the length of the second cable 2630 of the connection cord device 2600 interconnecting two adjacent second shoe care devices 1000 may be made smaller than the horizontal width L3 of the second shoe care device 1000, and electrical connection of multiple shoe care devices is made easily and neatly.

In an embodiment, the inlet unit 2100 and the outlet unit 2200 may be configured to be arranged along the vertical direction on the rear surfaces of the second shoe care devices 1000 (see FIG. 11C). In addition, the inlet units 2100 and the outlet units 2200 may be provided on the left or right sides of the second shoe care devices 1000.

In the shoe care system 2000 according to an embodiment of the present disclosure, each shoe care device 1 or 1000 has an upper surface and a lower surface which may be the same in size and shape on a plan view (when viewed in the third direction Z). The overall shape of the upper and lower surfaces of each shoe care device 1 and 1000 may be parallel to the horizontal direction or may be substantially parallel to the horizontal direction.

In an embodiment, the upper surface US of the first shoe care device 1 and the lower surface LS of the second shoe care device 1000 may be the same or substantially the same in size and shape.

In the shoe care system 2000 according to an embodiment of the present disclosure, the first shoe care device 1 may have a first height L1, and the second shoe care device 1000 may have a second height L2. In an embodiment, the horizontal width of the first shoe care device 1 may be the same or substantially the same as the horizontal width of the second shoe care device 1000. In an embodiment, the rear surface of the first shoe care device 1 and the rear surface of the second shoe care device 1000 may have the same horizontal width L3.

When the first height L1 of the first shoe care device 1 is greater than the second height L2 of the second shoe care device 1000, the first shoe care device 1 and the second shoe care device 1000 may be stacked such that the lower surface LS of the second shoe care device 1000 is in close contact with the upper surface US of the first shoe care device 1.

In this case, the outlet unit 2200 of the first shoe care device 1 may be provided to be biased on the upper side, and the inlet unit 2100 of the second shoe care device 1000 may be provided to be biased on the lower side. Accordingly, the length of the second cable 2630 of the connection cord device 2600 interconnecting the first shoe care device 1 and the second shoe care device 1000 may be made relatively short, and electrical connection between the first shoe care device 1 and the second shoe care device 1000 may be made easily and neatly.

In an embodiment, the upper surface US of the first shoe care device 1 and the lower surface LS of the second shoe care device 1000 may be configured to correspond to each other. In an embodiment, the upper surface US of the second shoe care device 1000 and the lower surface LS of the second shoe care device 1000 may be configured to correspond to each other. In an embodiment, the upper surface US of the first shoe care device 1 and the upper surface US of the second shoe care device 1000 may be the same or substantially the same in size and shape.

The description that the upper surface US and the lower surface LS correspond to each other may mean that the upper surface US and the lower surface LS are configured such that the relative movement in the horizontal direction between the shoe care devices 1 and 1000 is prevented.

When the upper surface US and the lower surface LS correspond to each other, one of the upper surface US and the lower surface LS may have a concave surface and the other may have a convex surface. In this case, the convex surface and the concave surface may be configured to be in contact with each other.

When the upper surface US and the lower surface LS correspond to each other, the edge E1 of the upper surface US may be configured such that its edge E1 protrudes more upward than the central portion as a whole, and the lower surface LS may be configured such that its edge E2 protrudes less downward than the central portion as a whole. In an embodiment, when a plurality of second shoe care devices 1000 are stacked vertically, the lower portion of a second shoe care device 1000 located on the upper side may be engaged with the upper edge E1 of a second shoe care device 1000 located on the lower side, and horizontal relative movement between the plurality of second shoe care devices 1000 can be prevented (see FIG. 11A).

When the plurality of second shoe care devices 1000 are stacked vertically, the length of the second cables 2630 of the connection cord devices 2600 interconnecting the plurality of second shoe care devices 1000 may be made greater than the height L2 of the second shoe care devices 1000 and smaller than twice the height L2, or may be made greater than the height L2 of the second shoe care devices 1000 and smaller than twice the width L3 of the second shoe care devices 1000. Considering trigonometric functions, the length of the second cables 2630 of the connection cord devices 2600 may be about 1.414*L2 or less (however, not less than L2) when L2 is greater than or equal to L3, or may be about 1.414*L3 or less (however, not less than L3) when L3 is greater than or equal to L2.

As described above, according to an embodiment of the present invention, power can be supplied to two or more shoe care devices, each shoe care device can be effectively used, and electrical connection of two or more shoe care devices can be made easily and neatly.

The inlet units 2100 are exposed from one sides of the outer cabinets 20 or 1101. That is, in the state in which the electrical cables (e.g., the power cord devices 2500 or the connection cord devices 2600) may be provided to be visible from the outside of the shoe care devices 2100 without separate components to hide or cover the inlet units 2100.

The outlet units 2200 are coupled to one sides of the outer cabinets 20 or 1101.

The outlet units 2200 may not to be exposed to the outside of the shoe care devices. That is, even in the state in which the electric cables (e.g., the connection cord devices 2600) are not coupled to the outlet units 2200, separate components 2300 may be provided to hide or cover the outlet units 2200 such that the outlet units 2200 are hidden from the outside of the shoe care devices.

To this end, the shoe care devices include flaps 2300. The flaps 2300 are provided in a substantially plate-like shape.

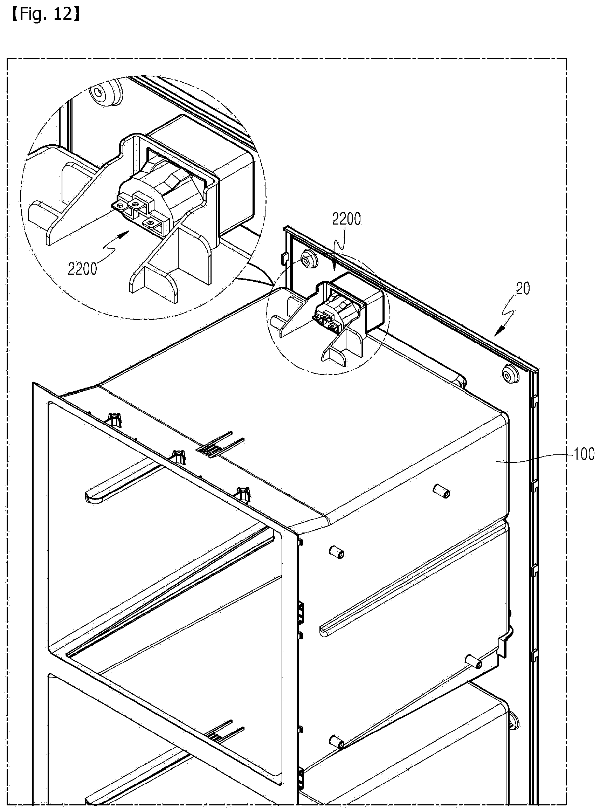

FIG. 12 is a view illustrating the internal appearance of an outer cabinet 20 in a shoe care device according to an embodiment of the present invention in the state in which an outlet unit 2200 is installed.

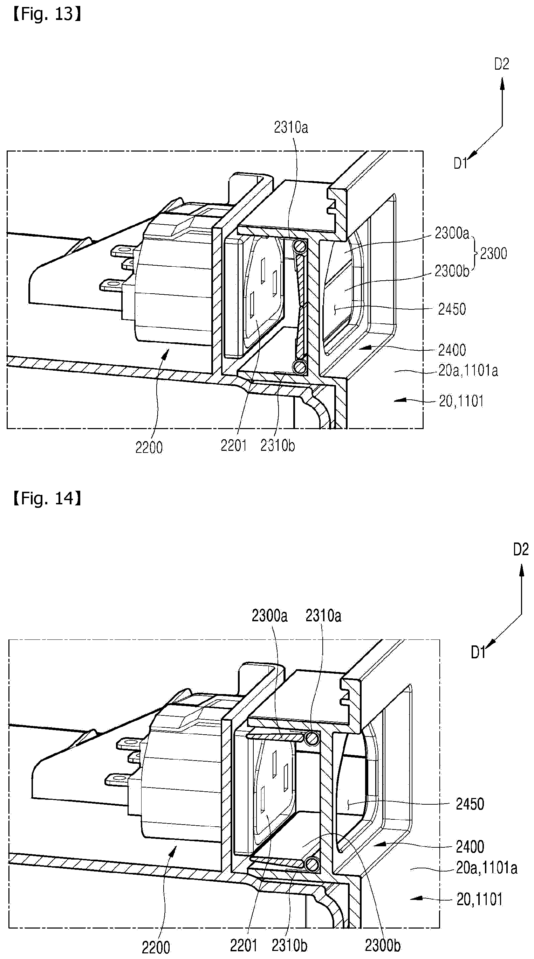

FIG. 13 is a view illustrating a cross-sectional shape of a portion of a shoe care device according to an embodiment of the present invention, in which an outer cabinet 20, an outlet unit 2200, and a flap 2300 are illustrated.

FIG. 14 is a view illustrating the flap 2300 in FIG. 13 in an open state.

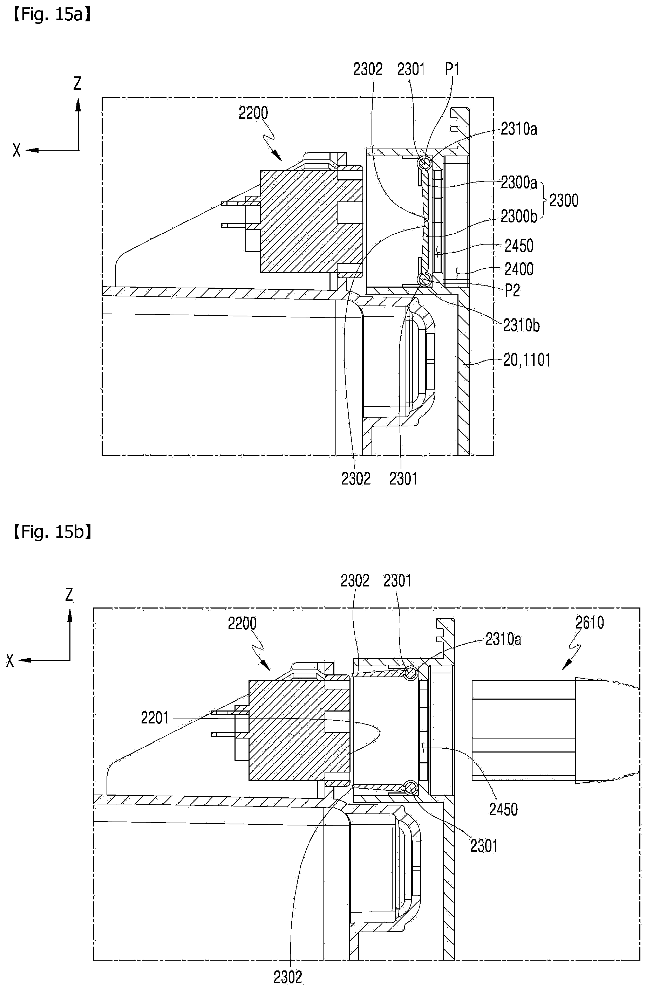

FIG. 15A is a cross-sectional view of a shoe care device in the state in which flaps 2300a and 2300b are closed.

FIG. 15B is a cross-sectional view of the shoe care device in the state in which the flaps 2300a and 2300b are opened. In FIG. 15B, the first connector 2610 is illustrated in the state of being spaced apart from the outlet unit 2200 to show each component. Of course, in practice, the first connector 2610 comes into contact with first and second flaps 2300a and 2300b, pushes the same to open, and is coupled to the outlet unit 2200.

In an embodiment of the present invention, the flap 2300 may include a first flap 2300a and a second flap 2300b.

The flap 2300 is coupled to the outer cabinet 20 or 1101 and configured to shield or expose the outlet unit 2200 when viewed from the outside.

The shoe care device may include an outlet hole 2450.

The outlet hole 2450 is a hole that penetrates the outer cabinet 20 or 1101. The outlet unit 2200 may be exposed to the outside through the outlet hole 2450.

The outlet hole 2450 may be formed in various shapes, such as a polygonal shape, a circular shape, and an oval shape.

The outlet hole 2450 may be formed to correspond to the cross-sectional shape of the first connector 2610. The size of the outlet hole 2450 may correspond to the size of the cross section of the first connector 2610 such that the first connector 2610 can be inserted into the outlet hole.

The flap 2300 may be hinge-coupled to the outer cabinet 20 or 1101 at the edge of the outlet hole 2450.

The flap 2300 has a first end 2301 and a second end 2302. The first end 2301 and the second end 2302 are the opposite ends of the flap 2300.

The flap 2300 is located inside the outer cabinet 20 or 1101, and the first end 2301 (the portion forming hinge axis) of the flap 2300 may be hinge-coupled to the inner surface of the outer cabinet 20 or 1101.

The flap 2300 may be configured to shield the outlet hole 2450. To this end, the size (area) of the flap 2300 may be made greater than or at least equal to the size (area) of the outlet hole 2450. When the flap 2300 is divided into the first flap 2300a and the second flap 2300b, the total area (size) of the first flap 2300a and the second flap 2300b may be made greater than or equal to the size of the outlet hole 2450.

As the outer surface of the flap 2300 is pressed inward by the first connector 2610, the outlet hole 2450 may be opened.

The shoe care device may include flap springs 2310a and 2310b.

The flap springs 2310a and 2310b may be configured to elastically support the flap 2300 in a direction in which the flap 2300 shields the outlet hole 2450. The flap springs 2310a and 2310b may have various shapes within the range of providing elastic force to the flap 2300. The flap springs 2310a and 2310b may be formed in the form of coil springs, torsion springs, leaf springs, or the like.

As will be described later, the flap springs 2310a and 2310b may be divided into a first flap spring 2310a and a second flap spring 2310b.

The flap 2300 and flap springs 2310a and 2310b may be located inside the outer cabinets 20 or 1101.

Since the flap springs 2310a and 2310b elastically support the flap 2300, the flap 2300 shields the outlet hole 2450 and the edge of the flap 2300 is brought into close contact with the inner surface of the outer cabinet 20 or 1101 (see FIG. 13). When an external force acts on the outer surface of the flap 2300 inward, the flap 2300 rotates about the hinge axis and the outlet hole 2450 is opened (see FIG. 14).

In a shoe care device according to an embodiment of the present invention, the flap 2300 is configured to shield the outlet hole 2450, so that external moisture, water, or the like can be prevented from penetrating into the outlet unit 2200 and excellent waterproofing can be achieved.

When the shoe care device is tilted, for example, even when the upper side of the shoe care device is tilted forward, the outlet hole 2450 is completely shielded by the flap 2300 so that external moisture, water, or the like can be prevented from penetrating toward the outlet unit 2200 through the outlet hole 2450, and excellent waterproofing can be achieved.

As described above, the shoe care device may include a first flap 2300a, a second flap 2300b, a first flap spring 2310a, and a second flap spring 2310b.

The first flap 2300a, the second flap 2300b, the first flap spring 2310a, and the second flap spring 2310b may be located inside the outer cabinet 20 or 1101.

The outlet hole 2450 may be opened when the outer surface of each of the first flap 2300a and the second flap 2300b is pressed inward.

The first flap 2300a may be hinge-coupled to the outer cabinet 20 or 1101 at a first point P1 on the edge of the outlet hole 2450.

The second flap 2300b may be hinge-coupled to the outer cabinet 20 or 1101 at a second point P2 opposite to the first point P1 on the edge of the outlet hole 2450.

When the first point P1 is located on a relatively higher side, the second point P2 is located on a relatively lower side. When the first point P1 is located on the left, the second point P2 is located on the right.

The first flap 2300a and the second flap 2300b may have the same configuration or similar configurations.

The first flap 2300a and the second flap 2300b may be rotationally symmetrical with respect to the center of the outlet hole 2450.

The first flap spring 2310a may be configured to elastically support the first flap 2300a in a direction in which the first flap 2300a shields the outlet hole 2450.

The second flap spring 2310b may be configured to elastically support the second flap 2300b in a direction in which the second flap 2300b shields the outlet hole 2450.

In the state in which no separate external force is applied to the first flap 2300a and the second flap 2300b, the first flap 2300a and the second flap 2300b shield the outlet hole 2450. At this time, the second end 2302 (the end opposite to the hinge axis) of the first flap 2300a and the second end 2302 (the end opposite to the hinge axis) of the second flap 2300b may be in close contact with each other or may be located very close to each other. That is, there may be no or almost no gap between the first flap 2300a and the second flap 2300b, and the first flap 2300a and the second flap 2300b may be configured to completely shield or almost completely shield the outlet hole 2450.

When an external force acts on the first flap spring 2310a and the second flap spring 2310b in the direction in which the elastic force increases in the first flap spring 2300a and the second flap spring 2300b, the first flap spring 2300a and the second flap spring 2310b rotate around hinges to open the outlet hole 2450. When the external force applied to the first flap 2300a and the second flap 2300b is removed, the first flap 2300a and the second flap 2300b are rotated in the opposite directions by the first flap spring 2310a and the second flap spring 2310b to close the outlet hole 2450.

In an embodiment of the present invention, one outlet hole 2450 may be shielded by one flap. That is, in an embodiment, one flap may be provided to shield the outlet unit 2200 (first embodiment).

In addition, in an embodiment of the present invention, one outlet hole 2450 may be shielded by two flaps 2300a and 2300b. That is, in an embodiment, a first flap 2300a and a second flap 2300b may be provided to shield the outlet unit 2200 (second embodiment).

Compared to the first embodiment, with the configuration of the second embodiment, the length from the first end 2301 to the second end 2302 in each of the first flap 2300a and the second flap 2300b may be made relatively short, and the length from the outlet hole 2450 to the outlet unit 2200 (the length from the outlet hole 2450 to the end surface 2201 of the outlet unit 2200) may be made relatively short.

In this case (second embodiment), the extent (length) in which the first connector 2610 passes through the outlet hole 2450 and enters the inside of the outer cabinet 20 or 1101 may be made relatively short, and the coupling between the first connector 2610 and the outlet unit 2200 is easily achieved.

In the outlet unit 2200, the width in a first width direction D1 may be made longer than the width in a second width direction D2 orthogonal to the first width direction D1. In this case, the first point P1 and the second point P2 may be spaced apart from each other along the second width direction D2.

In an embodiment of the present invention, when the first width direction D1 is the horizontal direction and the second width direction D2 is the vertical direction, and when the width of the outlet unit 2200 in the horizontal direction is greater than the width in the vertical direction, it may be possible to assume a case where the first point P1 and the second point P2 are spaced apart from each other along the horizontal direction, and the rotation axis of the first flap 2300a and the rotation axis of the second flap 2300b are parallel to the vertical direction (third embodiment).

Meanwhile, in an embodiment of the present invention, when the first width direction D1 is a horizontal direction and the second width direction D2 is the vertical direction, and when the width of the outlet unit 2200 in the horizontal direction is greater than the width in the vertical direction, the first point P1 and the second point P2 may be spaced apart from each other along the vertical direction. In this case, the first flap 2300a may be coupled to the upper side of the outlet hole 2450, and the second flap 2300b may be coupled to the lower side of the outlet hole 2450. In addition, at this time, the rotation axis of the first flap 2300a and the rotation axis of the second flap 2300b are parallel to the horizontal direction (fourth embodiment) (see FIGS. 13 and 14).

Compared to the third embodiment, with the configuration of the fourth embodiment, the length from the first end 2301 to the second end 2302 in each of the first flap 2300a and the second flap 2300b may be made relatively short, and the length from the outlet hole 2450 to the outlet unit 2200 may be made relatively shorter.

In this case (fourth embodiment), compared to the third embodiment, the extent (length) in which the first connector 2610 passes through the outlet hole 2450 and enters the inside of the outer cabinet 20 or 1101 may be made relatively short, and the coupling between the first connector 2610 and the outlet unit 2200 is easily achieved.

The outer cabinet 20 or 1101 may include a depression 2400.

The depression 2400 is recessed inward from the outer surface of the outer cabinet 20 or 1101.

The outlet hole 2450 may be provided in the depression 2400, and the outlet unit 2200 may be provided inside the depression 2400 (inside the outer cabinet 20 or 1101 at the point where the depression 2400 is provided). Accordingly, a stepped ledge is provided at the edge of the depression 2400.

By providing the stepped ledge in this way, even when external moisture or the like flows along the outer surface of the outer cabinet 20 or 1101, the possibility of moisture or the like flowing in through the outlet hole 2450 can be reduced, and it is possible to provide a structure that is advantageous for waterproofing.

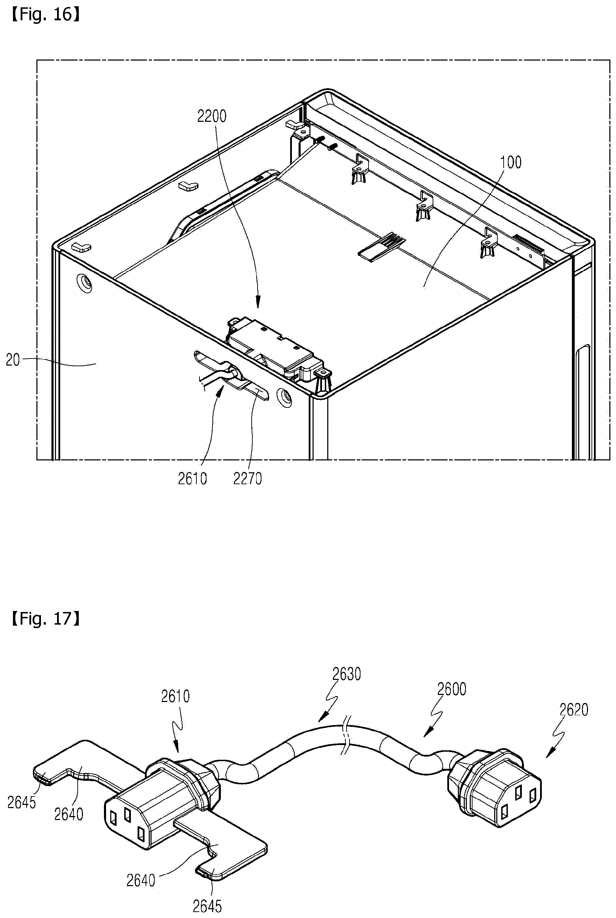

FIG. 16 is a view illustrating the internal appearance of an outer cabinet 20 in a shoe care device according to an embodiment of the present invention in the state in which a first connector 2610 and an outlet 2200 are coupled.

FIG. 17 is a view illustrating a connection cord device 2600 according to an embodiment of the present invention.

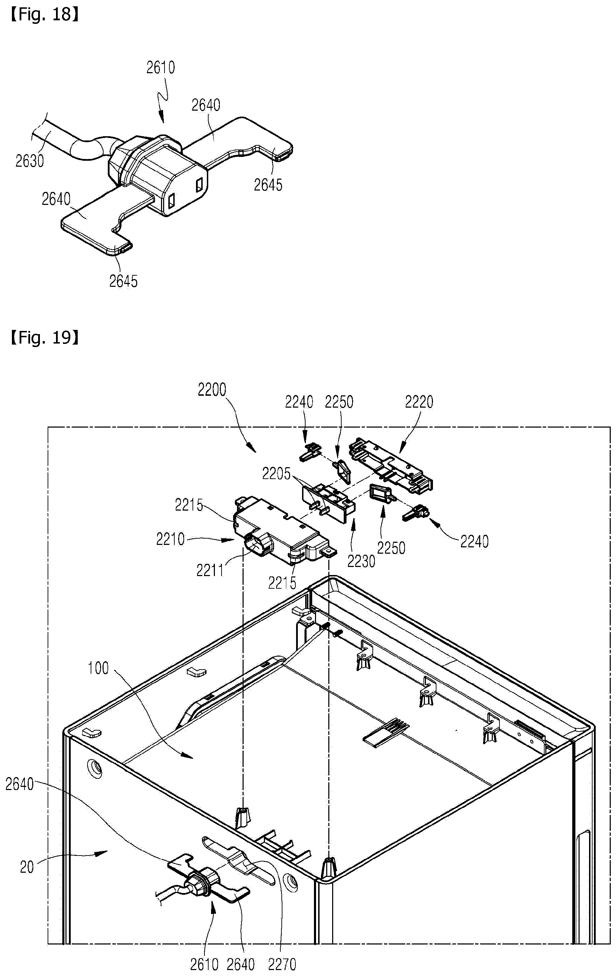

FIG. 18 is a view illustrating a first connector 2610 of the connection cord device 2600 according to an embodiment of the present invention.

FIG. 19 is a view illustrating the first connector 2610 and the outlet unit 2200 of FIG. 16 in a separated state. In FIG. 19, each component of the outlet unit 2200 is shown in a disassembled state.

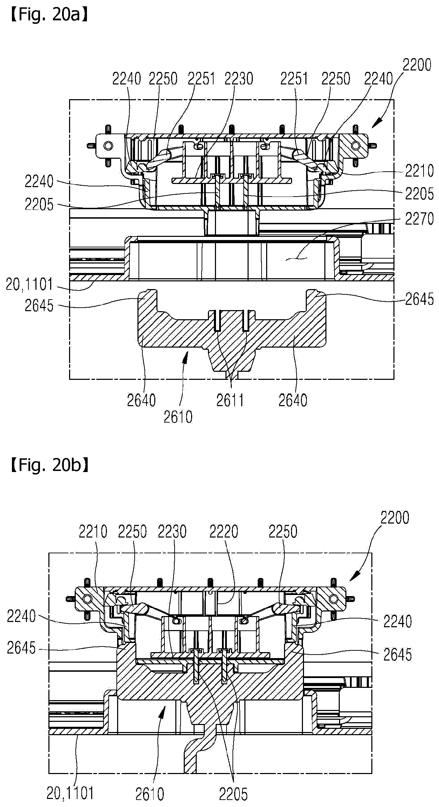

FIG. 20A is a cross-sectional view illustrating the first connector 2610 and the outlet unit 2200 in the state of being separated from a shoe care device according to an embodiment of the present invention.

FIG. 20B is a cross-sectional view illustrating the first connector 2610 and the outlet unit 2200 of FIG. 20A in the coupled state.

In the shoe care device according to an embodiment of the present invention, when the first connector 2610 is coupled to the outlet unit 2200, pins 2205 of the outlet unit 2200 move outward to connect the first connector 2610 to the outside to be electrically connected to terminals 2611.

The outer cabinet 20 or 1101 may include a connector slot 2270.

The connector slot 2270 is a hole formed through the outer cabinet 20 or 1101 to allow the first connector 2610 to be inserted.

The first connector 2610 may include a push rib 2640.

The push rib 2640 extends outward from the first connector 2610.

The push rib 2640 may protrude from the center of the first connector 2610 to opposite sides. A pair of push ribs 2640 may be provided to be symmetrical with respect to the center of the first connector 2610.

The push ribs 2640 may be provided in a plate shape.

The push ribs 2640 may each include a rib end 2645. The rib end 2645 may protrude further than the first connector 2610 in a distal direction (the direction in which the rib end is fastened to the outlet unit 2200).

The outlet unit 2200 may include a fixed bracket 2220, a movable bracket 2230, a slider 2240, and a lever 2250. The outlet unit 2200 may include an outlet housing 2210.

The outlet housing 2210 may provide the entire body of the outlet unit 2200, and components of the outlet unit 2200 may be coupled to the outlet housing 2210.

The outlet housing 2210 is located inside the outer cabinet 20 or 1101.

In the shoe care device, the position of the outlet housing 2210 may be fixed. The outlet housing 2210 may be fixed to a frame of the shoe care device, or may be fixed to the inner cabinet 100 of the shoe care device.

The outlet housing 2210 is configured to accommodate a fixed bracket 2220, a movable bracket 2230, a slider 2240, and a lever 2250.

The outlet housing 2210 is provided with a housing slit 2215 into which a push rib 2640 is inserted. The housing slit 2215 may be provided on each of the left and right sides of the outlet housing 2210 to be bilaterally symmetrical with respect to the center of the outlet housing 2210. When the rib end 2645 of the push rib 2640 is inserted into the housing slit 2215, the rib end 2645 may press and move the slider 2240.

The outlet housing 2210 is provided with an outlet opening 2211 into which the central portion of the first connector 2610 is inserted.

The fixed bracket 2220 is located inside the outer cabinet 20 or 1101. The fixed bracket 2220 may be located inside the outlet housing 2210 and fixed to the outlet housing 2210. The fixed bracket 2220 may be configured integrally with a portion of the outlet housing 2210.

The pin 2205 (pin through which electrical connection is made) of the outlet unit 2200 is fixed to the movable bracket 2230. The movable bracket 2230 is movable relative to the fixed bracket 2220. The movable bracket 2230 is coupled to the fixed bracket 2220 to be reciprocally movable inward and outward relative to the fixed bracket 2220 (to the inside and outside of the outer cabinet 20 or 1101, for example, in a direction parallel to the first direction or the second direction).

The slider 2240 configured to be moved by being pressed by the push rib 2640. The slider 2240 is movable relative to the fixed bracket 2220. The slider 2240 is coupled to the fixed bracket 2220 to be reciprocally movable inward and outward relative to the fixed bracket 2220 (to the inside and outside of the outer cabinet 20 or 1101).

The lever 2250 is rotatably coupled to the fixed bracket 2220. The rotation axis 2251 of the lever 2250 may be parallel to the third direction Z. Opposite ends of the lever 2250 centered on the rotation axis 2251 of the lever 2250 are coupled to the movable bracket 2230 and the slider 2240, respectively.

Therefore, when the slider 2240 moves inward (to the inside of the outer cabinet 20 or 1101), the movable bracket 2230 moves outward (to the outside of the outer cabinet 20 or 1101), and when the slider 2240 moves outward (to the outside of the outer cabinet 20 or 1101), the movable bracket 2230 moves inward (to the inside of the outer cabinet 20 or 1101).

In the outlet unit 2200, a pair of sliders 2240 and a pair of levers 2250 may be provided to be symmetrical with respect to the center of the outlet unit 2200.

The outlet unit 2200 may include a spring.

The spring of the outlet unit 2200 may elastically support the movable bracket 2230 such that the movable bracket 2230 moves inward (to the inside of the outer cabinet 20 or 1101). The spring of the outlet unit 2200 may have various spring structures.

The spring of the outlet unit 2200 may elastically support the slider 2240 such that the slider 2240 moves outward (to the inside of the outer cabinet 20 or 1101).

When the first connector 2610 is not coupled to the outlet unit 2200, the pin 2205 of the outlet unit 2200 may be located inside the outlet housing 2210.

When the first connector 2610 is coupled to the outlet unit 2200, the pin 2205 of the outlet unit 2200 may penetrate the outlet housing 2210 and protrude out of the outlet housing 2210.

By the above-described configuration, the possibility of external moisture, water, or the like flowing in toward the outlet unit 2200 and the possibility of external moisture, water, or the like flowing in toward the fin 2205 of the outlet unit 2200 can be significantly reduced, and it is possible to configure a shoe care device with a structure that is advantageous for waterproofing.

### [Industrial Applicability]

According to an embodiment of the present invention, power can be supplied to two or more shoe care devices via one power cord device, and electrical connection of two or more shoe care devices can be made easily and neatly.

## Claims

1. A shoe care device comprising:
an accommodation space (101, 1010) configured to accommodate shoes;
an outer cabinet (20, 1101) defining an exterior of the shoe care device;
an operation unit (2700) configured to adjust one or more of temperature, humidity, and air flow in the accommodation space (101, 1010);
a power supply unit (11, 1601) configured to supply power to the operation unit (2700);
an inlet unit (2100) electrically connected to the power supply unit (11, 1601) and configured such that external power enters therethrough; and
an outlet unit (2200) electrically connected to the power supply unit (11, 1601) and configured such that power goes out therethrough,
**characterized in that** the inlet unit (2100) is exposed on one side of the outer cabinet (20, 1101), and
wherein the shoe care device further comprises:
a flap (2300) coupled to the outer cabinet (20, 1101) and configured to shield or expose the outlet unit (2200).

2. The shoe care device of claim 1, wherein the inlet unit (2100) and the outlet unit (2200) are provided on a rear surface of the outer cabinet (20, 1101).

3. The shoe care device of claim 1 or 2, further comprising:
an outlet hole (2450) penetrating the outer cabinet (20, 1101), wherein the outlet unit (2200) is exposed through the outlet hole (2450), wherein the flap (2300) is hinge-coupled to the outer cabinet (20, 1101) at an edge of the outlet hole (2450); and
a flap spring (2310a, 2310b) configured to elastically support the flap (2300) in a direction in which the flap (2300) shields the outlet hole (2450),
wherein, preferably, the flap (2300) and the flap spring (2310a, 2310b) are located inside the outer cabinet (20, 1101), and wherein the outlet hole (2450) is opened when the outer surface of the flap (2300) is pressed inward.

4. The shoe care device of claim 1 or 2,
wherein the flap (2300) comprises a first flap (2300a) and a second flap (2300b), and
wherein the shoe care device further comprising:
an outlet hole (2450) penetrating the outer cabinet (20, 1101), wherein the outlet unit (2200) is exposed through the outlet hole (2450); and
the first flap (2300a) hinge-coupled to the outer cabinet (20, 1101) at a first point of the edge of the outlet hole (2450);
the second flap (2300b) hinge-coupled to the outer cabinet (20, 1101) at a second point of the edge of the outlet hole (2450) located opposite to the first point;
a first flap spring (2310a) configured to elastically support the first flap (2300a) in a direction in which the first flap (2300a) shields the outlet hole (2450); and
a second flap spring (2310b) configured to elastically support the second flap (2300b) in a direction in which the second flap (2300b) shields the outlet hole (2450).

5. The shoe care device of claim 4, wherein a width of the outlet unit (2200) in a first width direction is longer than a width of the outlet unit (2200) in a second width direction perpendicular to the first width direction, and wherein the first point and the second point are spaced apart from each other along the second width direction, and/or
wherein the first flap (2300a), the second flap (2300b), the first flap spring (2310a), and the second flap spring (2310b) are located inside the outer cabinet (20, 1101), and wherein the outlet hole (2450) is opened when each of outer surfaces of the first and second flaps (2300a, 2300b) is pressed inward.

6. The shoe care device of one of claims 1 or 3 to 5, wherein the outer cabinet (20, 1101) comprises a depression (2400) recessed inward from an outer surface of the outer cabinet (20, 1101), and
wherein the outlet unit (2200) is provided inside the outer cabinet (20, 1101) at a point where the depression (2400) is formed.

7. The shoe care device of one of claims 1 to 6, further comprising:
a power cord device (2500) comprising an inlet connector (2520) configured to be detachably attached to the inlet unit (2100); and
a connection cord device (2600) comprising a first connector (2610) configured to be detachably attached to the outlet unit (2200), a second connector (2620) configured to be detachably attached to the inlet unit (2100), and a cable electrically interconnecting the first connector (2610) and the second connector (2620),
wherein the first connector (2610) is coupled to the outlet unit (2200), but is not coupled to the inlet unit (2100).

8. The shoe care device of one of claims 1 to 7, further comprising:
a connection cord device (2600) comprising a first connector (2610) configured to be detachably attached to the outlet unit (2200), a second connector (2620) configured to be detachably attached to the inlet unit (2100), and a cable electrically interconnecting the first connector (2610) and the second connector (2620),
wherein, when the first connector (2610) is coupled to the outlet unit (2200), a pin of the outlet unit (2200) moves outward to be electrically connected to a terminal of the first connector (2610).

9. The shoe care device of claim 8, wherein the first connector (2610) comprises an outwardly extending push rib (2640), and
wherein the outlet unit (2200) comprises:
a fixed bracket (2220) located inside the outer cabinet (20, 1101);
a movable bracket (2230) to which the pin of the outlet unit (2200) is fixed and which is configured to be movable relative to the fixed bracket (2220);
a slider (2240) configured to move while being pressed by the push rib (2640);
and
a lever (2250) rotatably coupled to the fixed bracket (2220), and having opposite ends coupled to the movable bracket (2230) and the slider (2240), respectively.

10. The shoe care device of claim 9, wherein a pair of push ribs (2640) are provided and arranged symmetrically with respect to a center of the first connector (2610), and wherein a pair of sliders (2240) and a pair of lever (2250) are provided and arranged symmetrically with respect to a center of the outlet unit (2200), and/or
wherein the outlet unit (2200) further comprises an outlet housing (2210) configured to accommodate the fixed bracket (2220), the movable bracket (2230), the slider (2240), and the lever (2250), and is provided with a housing slit (2215) into which the push rib (2640) is inserted, and/or
wherein the outer cabinet (20, 1101) comprises a connector slot (2270) through which the first connector (2610) is inserted.

11. The shoe care device of claim 10, wherein, when the first connector (2610) is not coupled to the outlet unit (2200), the pin of the outlet unit (2200) is located inside the outlet housing (2210), and
wherein, when the first connector (2610) is coupled to the outlet unit (2200), the pin of the outlet unit (2200) penetrates the outlet housing (2210) and protrudes outside the outlet housing (2210).

## Patentansprüche

1. Schuhpflegevorrichtung, umfassend:
einen Aufnahmeraum (101, 1010), der in der dazu eingerichtet ist, Schuhe aufzunehmen;
ein Außengehäuse (20, 1101), das die Außenseite der Schuhpflegevorrichtung bildet;
eine Betriebseinheit (2700), die dazu eingerichtet ist, Temperatur, Luftfeuchtigkeit und Luftstrom in dem Aufnahmeraum (101, 1010) einzustellen;
eine Stromversorgungseinheit (11, 1601), die dazu eingerichtet ist, die Betriebseinheit (2700) mit Strom zu versorgen;
eine Eingangseinheit (2100), die elektrisch mit der Stromversorgungseinheit (11, 1601) verbunden ist und dazu eingerichtet ist, externe Energie durch sie hindurch zu führen; und
eine Ausgangseinheit (2200), die elektrisch mit der Stromversorgungseinheit (11, 1601) verbunden ist und dazu eingerichtet ist, Energie durch sie hindurch ausgegeben wird,
**dadurch gekennzeichnet, dass**
die Eingangseinheit (2100) an einer Seite des Außengehäuses (20, 1101) freiliegt, und
wobei die Schuhpflegevorrichtung ferner umfasst:
eine Klappe (2300), die mit dem Außengehäuse (20, 1101) verbunden ist und dazu eingerichtet ist, die Ausgabeeinheit (2200) abzuschirmen oder freizugeben.

2. Schuhpflegevorrichtung nach Anspruch 1, wobei die Eingangseinheit (2100) und die Ausgangseinheit (2200) an einer Rückseite des Außengehäuses (20, 1101) vorgesehen sind.

3. Schuhpflegevorrichtung nach Anspruch 1 oder 2, ferner umfassend:
eine das Außengehäuse (20, 1101) durchdringende Auslassöffnung (2450), wobei die Ausgangseinheit (2200) durch die Auslassöffnung (2450) freigelegt ist, wobei die Klappe (2300) an einer Kante der Auslassöffnung (2450) schwenkbar mit dem Außengehäuse (20, 1101) verbunden ist; und
eine Klappenfeder (2310a, 2310b), die dazu eingerichtet ist, die Klappe (2300) elastisch in einer Richtung abzustützen, in der die Klappe (2300) die Auslassöffnung (2450) abschirmt,
wobei vorzugsweise die Klappe (2300) und die Klappenfeder (2310a, 2310b) im Inneren des Außengehäuses (20, 1101) angeordnet sind und wobei die Auslassöffnung (2450) geöffnet wird, wenn die Außenfläche der Klappe (2300) nach innen gedrückt wird.

4. Schuhpflegevorrichtung nach Anspruch 1 oder 2,
wobei die Klappe (2300) eine erste Klappe (2300a) und eine zweite Klappe (2300b) umfasst, und
wobei die Schuhpflegevorrichtung ferner umfasst:
eine Auslassöffnung (2450), die das Außengehäuse (20, 1101) durchdringt, wobei die Ausgangseinheit (2200) durch die Auslassöffnung (2450) freigelegt ist; und
die erste Klappe (2300a) an einem ersten Punkt am Rand der Auslassöffnung (2450) schwenkbar mit dem Außengehäuse (20, 1101) verbunden ist;
wobei die zweite Klappe (2300b) an einem zweiten Punkt am Rand der Auslassöffnung (2450), der dem ersten Punkt gegenüberliegt, schwenkbar mit dem Außengehäuse (20, 1101) verbunden ist;
eine erste Klappenfeder (2310a), die dazu eingerichtet ist, die erste Klappe (2300a) in einer Richtung elastisch abzustützen, in der die erste Klappe (2300a) die Auslassöffnung (2450) abschirmt; und
eine zweite Klappenfeder (2310b), die dazu eingerichtet ist, die zweite Klappe (2300b) in einer Richtung elastisch abzustützen, in der die zweite Klappe (2300b) die Auslassöffnung (2450) abschirmt.

5. Schuhpflegevorrichtung nach Anspruch 4, wobei eine Breite der Auslassvorrichtung (2200) in einer ersten Breitenrichtung größer ist als eine Breite der Auslassvorrichtung (2200) in einer zweiten Breitenrichtung senkrecht zur ersten Breitenrichtung, und wobei der erste Punkt und der zweite Punkt entlang der zweiten Breitenrichtung voneinander beabstandet sind, und/oder
wobei die erste Klappe (2300a), die zweite Klappe (2300b), die erste Klappenfeder (2310a) und die zweite Klappenfeder (2310b) sich im Inneren des Außengehäuses (20, 1101) befinden, und wobei die Auslassöffnung (2450) geöffnet wird, wenn ich jede der Außenflächen der ersten und zweiten Klappen (2300a, 2300b) nach innen gedrückt werden.

6. Schuhpflegevorrichtung nach einem der Ansprüche 1 oder 3 bis 5, wobei das Außengehäuse (20, 1101) eine Vertiefung (2400) aufweist, die von einer Außenfläche des Außengehäuses (20, 1101) nach innen versetzt ist, und
wobei die Ausgangseinheit (2200) innerhalb des Außengehäuses (20, 1101) an einer Stelle vorgesehen ist, an der die Vertiefung (2400) ausgebildet ist.

7. Schuhpflegevorrichtung nach einem der Ansprüche 1 bis 6, ferner umfassend:
eine Netzkabelvorrichtung (2500), die einen Eingangsstecker (2520) umfasst, der dazu eingerichtet ist, lösbar an der Eingangseinheit (2100) befestigt zu werden; und
eine Verbindungskabelvorrichtung (2600), die einen ersten Stecker (2610), der dazu eingerichtet ist, lösbar an der Ausgangseinheit (2200) befestigt zu werden, einen zweiten Stecker (2620), der dazu eingerichtet ist, lösbar an der Eingangseinheit (2100) befestigt zu werden, und ein Kabel, das den ersten Stecker (2610) und den zweiten Stecker (2620) elektrisch miteinander verbindet, umfasst,
wobei der erste Stecker (2610) mit der Ausgangseinheit (2200) verbunden ist, jedoch nicht mit der Eingangseinheit (2100) verbunden ist.

8. Schuhpflegevorrichtung nach einem der Ansprüche 1 bis 7, ferner umfassend:
eine Verbindungskabelvorrichtung (2600), die einen ersten Stecker (2610), der dazu eingerichtet ist, lösbar an der Ausgangseinheit (2200) befestigt zu werden, einen zweiten Stecker (2620), der dazu eingerichtet ist, lösbar an der Eingangseinheit (2100) befestigt zu werden, und ein Kabel, das den ersten Stecker (2610) und den zweiten Stecker (2620) elektrisch miteinander verbindet, umfasst,
wobei, wenn der erste Stecker (2610) mit der Ausgangseinheit (2200) gekoppelt ist, sich ein Stift der Ausgangseinheit (2200) nach außen bewegt, um elektrisch mit einem Anschluss des ersten Steckers (2610) verbunden zu werden.

9. Schuhpflegevorrichtung nach Anspruch 8, wobei der erste Stecker (2610) eine sich nach außen erstreckende Druckrippe (2640) umfasst, und
wobei die Ausgangseinheit (2200) umfasst:
eine feste Halterung (2220), die sich im Inneren des Außengehäuses (20, 1101) befindet;
eine bewegliche Halterung (2230), an der der Stift der Ausgangseinheit (2200) befestigt ist und die dazu eingerichtet ist, relativ zur festen Halterung (2220) beweglich zu sein;
einen Schieber (2240), der dazu eingerichtet ist, sich zu bewegen, während er von der Druckrippe (2640) gedrückt wird; und
einen Hebel (2250), der drehbar mit der feststehenden Halterung (2220) verbunden ist und dessen gegenüberliegende Enden jeweils mit der beweglichen Halterung (2230) und dem Schieber (2240) verbunden sind.

10. Schuhpflegevorrichtung nach Anspruch 9, wobei ein Paar Druckrippen (2640) vorgesehen und symmetrisch in Bezug auf eine Mitte des ersten Steckers (2610) angeordnet ist, und wobei ein Paar Schieber (2240) und ein Paar Hebel (2250) vorgesehen und symmetrisch in Bezug auf eine Mitte der Ausgabeeinheit (2200) angeordnet sind, und/oder
wobei die Ausgangseinheit (2200) ferner ein Ausgangsgehäuse (2210) umfasst, das dazu eingerichtet ist, die feste Halterung (2220), die bewegliche Halterung (2230), den Schieber (2240) und den Hebel (2250) aufzunehmen, und mit einem Gehäuseschlitz (2215) versehen ist, in den die Druckrippe (2640) eingeführt ist, und/oder
wobei das Außengehäuse (20, 1101) einen Steckerschlitz (2270) umfasst, durch den der erste Stecker (2610) eingeführt wird.

11. Schuhpflegevorrichtung nach Anspruch 10, wobei, wenn der erste Stecker (2610) nicht mit der Ausgangseinheit (2200) gekoppelt ist, sich der Stift der Ausgangseinheit (2200) innerhalb des Ausgangsgehäuses (2210) befindet, und
wobei, wenn der erste Stecker (2610) mit der Ausgangseinheit (2200) verbunden ist, der Stift der Ausgangseinheit (2200) das Ausgangsgehäuse (2210) durchdringt und aus dem Ausgangsgehäuse (2210) herausragt.

## Revendications

1. Dispositif d'entretien de chaussures comprenant :
un espace de logement (101, 1010) conçu pour recevoir des chaussures;
un placard extérieur (20, 1101) délimitant la partie extérieure du dispositif d'entretien de chaussures;
une unité de commande (2700) conçue pour réguler l'une parmi la température, l'humidité et/ou le débit d'air dans l'espace de logement (101, 1010);
un bloc d'alimentation (11, 1601) conçu pour alimenter l'unité de commande (2700);
une unité d'entrée (2100) connectée électriquement au bloc d'alimentation (11, 1601) et configurée de manière à permettre l'entrée d'une alimentation externe; et
une unité de sortie (2200) connectée électriquement au bloc d'alimentation (11, 1601) et configurée de manière à permettre la sortie d'une alimentation; et
**caractérisé en ce que**
l'unité d'entrée (2100) est visible sur l'un des côtés du placard extérieur (20, 1101), et
dans lequel le dispositif d'entretien de chaussures comprend:
un volet (2300) relié au placard extérieur (20, 1101) et conçu pour protéger ou rendre visible l'unité de sortie (2200).

2. Dispositif d'entretien de chaussures selon la revendication 1, dans lequel l'unité d'entrée (2100) et l'unité de sortie (2200) sont disposées sur une face arrière du placard extérieur (20, 1101).

3. Dispositif d'entretien de chaussures selon la revendication 1 à 2, comprenant en outre:
un orifice de sortie (2450) traversant le placard extérieur (20, 1101), dans lequel l'unité de sortie (2200) est visible par l'orifice de sortie (2450), dans lequel le volet (2300) est articulé sur le placard extérieur (20, 1101) au niveau d'un bord de l'orifice de sortie (2450); et
un ressort de volet (2310a, 2310b) conçu pour soutenir élastiquement le clapet (2300) dans une direction dans laquelle le volet recouvre l'orifice de sortie (2450),
dans lequel, de préférence, le volet (2300) et le ressort de volet (2310a, 2310b) sont situés à l'intérieur du placard extérieur (20, 1101), et dans lequel l'orifice de sortie (2450) s'ouvre lorsque la surface extérieure du volet (2300) est enfoncée vers l'intérieur.

4. Dispositif d'entretien de chaussures selon la revendication 1 ou 2,
dans lequel le volet (2300) comprend un premier volet (2300a) et un deuxième volet (2300b), et
dans lequel le dispositif d'entretien de chaussures comprend:
un orifice de sortie (2450) traversant le placard extérieur (20, 1101), dans lequel l'unité de sortie (2200) est ainsi visible par l'orifice de sortie (2450) ; et
le premier volet (2300a) articulé sur le placard extérieur (20, 1101) en un premier point situé sur le bord de l'orifice de sortie (2450);
le deuxième volet (2300b) articulé sur le placard extérieur (20, 1101) en un deuxième point du bord de l'orifice de sortie (2450) situé à l'opposé du premier point;
un premier ressort de volet (2310a) conçu pour soutenir élastiquement le clapet (2300a) dans une direction dans laquelle le premier volet (2 »00a) protège l'orifice de sortie (2450); et
un deuxième ressort de volet (2310b) conçu pour soutenir élastiquement le clapet (2300b) dans une direction dans laquelle le deuxième volet (2300b) protège l'orifice de sortie (2450).

5. Dispositif d'entretien de chaussures selon la revendication 4, dans lequel la largeur de l'unité de sortie (2200) dans un premier sens de la largeur est supérieure à la largeur de l'unité de sortie (2200) dans un deuxième sens de la largeur perpendiculaire au premier sens de la largeur, et dans lequel le premier point et le deuxième point sont espacés l'un de l'autre le long du deuxième sens de la largeur, et/ou
dans lequel le premier volet (2300a), le deuxième volet (2300b), le premier ressort de volet (2310a) et le deuxième ressort de volet (2310b) sont situés à l'intérieur du placard extérieur (20, 1101), et dans lequel l'orifice de sortie (2450) s'ouvre lorsque chacune des surfaces extérieures des premier et deuxième volets (2300a, 2300b) est enfoncée vers l'intérieur.

6. Dispositif d'entretien de chaussures selon l'une quelconque des revendications 1 ou 3 à 5, dans lequel le placard extérieur (20, 1101) comprend un renfoncement (2400) situé en retrait par rapport à la surface extérieure du placard extérieur (20, 1101), et
dans lequel l'unité de sortie (2200) est disposée à l'intérieur du placard extérieur (20, 1101) à l'endroit où est formé le renfoncement (2400).

7. Dispositif d'entretien de chaussures selon l'une quelconque des revendications 1 à 6, comprenant en outre:
un dispositif de cordon d'alimentation (2500) comprenant un connecteur d'entrée (2520) conçu pour être fixé amovible à l'unité d'entrée (2100); et
un dispositif de cordon de raccordement (2600) comprenant un premier connecteur (2610) conçu pour être fixé amovible à l'unité de sortie (2200), un deuxième connecteur (2620) conçu pour être fixé amovible à l'unité d'entrée (2100), et un câble reliant électriquement le premier connecteur (2610) et le deuxième connecteur (2620),
dans lequel le premier connecteur (2610) est couplé à l'unité de sortie (2200) mais n'est pas couplé à l'unité d'entrée (2100).

8. Dispositif d'entretien de chaussures selon l'une quelconque des revendications 1 à 7, comprenant en outre:
un dispositif de cordon de raccordement (2600) comprenant un premier connecteur (2610) conçu pour être fixé amovible à l'unité de sortie (2200), un deuxième connecteur (2620) conçu pour être fixé amovible à l'unité d'entrée (2100), et un câble reliant électriquement le premier connecteur (2610) et le deuxième connecteur (2620),
dans lequel, lorsque le premier connecteur (2610) est couplé à l'unité de sortie (2200), une broche de l'unité de sortie (2200) se déplace vers l'extérieur pour être connectée électriquement à une borne du premier connecteur (2610).

9. Dispositif d'entretien de chaussures selon la revendication 8, dans lequel le premier connecteur (2610) comprend une nervure de poussée (2640) s'étendant vers l'extérieur, et
dans lequel l'unité de sortie (2200) comprend:
un support fixe (2220) situé à l'intérieur du placard extérieur (20, 1101);
un support mobile (2230) auquel est fixée la broche de l'unité de sortie (2200) et qui est conçu pour pouvoir se déplacer par rapport au support fixe (2220);
un coulisseau (2240) conçu pour se déplacer lorsqu'il est poussé par la nervure de poussée (2640); et
un levier (2250) relié en rotation au support fixe (2220), et dont les extrémités opposées sont reliées respectivement au support mobile (2230) et au coulisseau (2240).

10. Dispositif d'entretien de chaussures selon la revendication 9, dans lequel une paire de nervures de poussée (2640) est disposée symétriquement par rapport au centre du premier connecteur (2610), et dans lequel une paire de coulisseaux (2240) et une paire de leviers (2250) sont disposées symétriquement par rapport au centre de l'unité de sortie (2200), et/ou
dans lequel l'unité de sortie (2200) comprend en outre un boîtier de sortie (2210) configuré pour recevoir le support fixe (2220), le support mobile (2230), le coulisseau (2240) et le levier (2250), et est pourvu d'une fente de logement (2215) dans laquelle est insérée la nervure de poussée (2640), et/ou
dans lequel le placard extérieur (20, 1101) comporte une fente de connexion (2270) à travers laquelle le premier connecteur (2610) est inséré.

11. Dispositif d'entretien de chaussures selon la revendication 10, dans lequel le premier connecteur (2610) n'est pas raccordé à l'unité de sortie (2200), la broche de l'unité de sortie (2200) se trouve à l'intérieur du boîtier de sortie (2210), et
dans lequel, lorsque le premier connecteur (2610) est couplé à l'unité de sortie (2200), la broche de l'unité de sortie (2200) pénètre dans le boîtier de sortie (2210) et fait saillie à l'extérieur du boîtier de sortie (2210).
